# EUROPEAN PATENT APPLICATION

(11) **EP 1 978 105 A1**
(43) Date of publication of application: **08.10.2008**
(21) Application number: 07007072.7
(22) Date of filing: 04.04.2007
(51) Int. Cl.: C12Q 1/68

(54) **TBC1D1 as a marker in the response to dietary fat and obesity**

(71) Applicant: DEUTSCHES INSTITUT FÜR ERNÄHRUNGSFORSCHUNG - STIFTUNG DES ÖFFENTLICHEN RECHTS - VERTRETEN DURCH DEN STIFTUNGSVORSTAND, 14558 Nuthetal (DE)
(72) Inventor: Al-Hasani, Hadi, Dr., 14469 Potsdam (DE); Joost, Hans-Georg, Prof. Dr. Dr., 14532 Kleinmachnow (DE); Kluge, Reinhart, Dr., 14558 Nuthetal (DE); Leicht, Katja, 14473 Potsdam (DE)
(74) Representative: Krauss, Jan

(57) **Abstract**

The present invention relates to the gene TBC 1 D and its use as a genetic marker in the response to dietary fat, and obesity and diabetes. TBC1D1 provides a valuable tool both for diagnostic as well as therapeutic approaches, in order to treat or prevent obesity or diabetes, in particular in response to dietary fat.

## Description

The present invention relates to the gene TBC1D1 and its use as a genetic marker in the response to dietary fat, and obesity and diabetes. TBC1D1 provides a valuable tool both for diagnostic as well as therapeutic approaches, in order to treat or prevent obesity or diabetes, in particular in response to dietary fat.

### Description

The incidence of obesity has reached epidemic levels in the developed world (Hedley, A.A., Ogden, C.L., Johnson, C.L., Carroll, M.D., Curtin, L.R. and Flegal, K.M. (2004) Prevalence of overweight and obesity among US children, adolescents, and adults, 1999-2002. JAMA, 291, 2847-2850, Flegal, K.M., Carroll, M.D., Ogden, C.L. and Johnson, C.L. (2002) Prevalence and trends in obesity among US adults, 1999-2000. JAMA, 288, 1723-1727.). If unchecked, the rising rates of obesity (and its associated morbidities) pose a significant threat to human health and a costly burden to public healthcare systems. Unfortunately, patients with severe obesity [usually defined as having a body-mass index (BMI) of over 35] have very few medical options. In fact, with the exception of surgical intervention, there are no effective long-term therapies (Brolin, R.E. (2002) Bariatric surgery and long-term control of morbid obesity. JAMA, 288, 2793-2796). A general goal is to define the genetic etiology of obesity predisposition to enable the development of effective therapeutic interventions.

Obesity is a complex genetic disease. Although it is clearly heritable (Price, R.A., Ness, R. and Laskarzewski, P. (1990) Common major gene inheritance of extreme overweight. Hum. Biol., 62, 747-765. Hunt, S.C., Hasstedt, S.J., Kuida, H., Stults, B.M., Hopkins, P.H. and Williams, R.R. (1989) Genetic heritability and common environmental components of resting and stressed blood pressures, lipids, and body mass index in Utah pedigrees and twins. Am. J. Epidemiol., 129, 625-638. Bell, C.G., Walley, A.J. and Froguel, P. (2005) The genetics of human obesity. Nat. Rev. Genet., 6, 221-234.) (e.g. first-degree relatives of obese patients have up to a 9-fold increase in disease risk (Price, R.A. and Lee, J.H. (2001) Risk ratios for obesity in families of obese African-American and Caucasian women. Hum. Hered., 51, 35-40.), the segregation of the disease in obesity-prone families is non-Mendelian (Borecki, LB., Higgins, M., Schreiner, P.J., Arnett, D.K., Mayer-Davis, E., Hunt, S.C. and Province, M.A. (1998) Evidence for multiple determinants of the body mass index: the National Heart, Lung, and Blood Institute Family Heart Study. Obes. Res., 6, 107-114.). Also, many studies have provided evidence to suggest that risk for obesity is determined not only by specific genotypes but also by significant gene/gene and gene/environment interactions (Dong, C., Wang, S., Li, W.D., Li, D., Zhao, H. and Price, R.A. (2003) Interacting genetic loci on chromosomes 20 and 10 influence extreme human obesity. Am. J. Hum. Genet., 72, 115-124. Dong, C., Li, W.D., Li, D. and Price, R.A. (2005) Interaction between obesity-susceptibility loci in chromosome regions 2p25-p24 and 13q13-q21. Eur. J. Hum. Genet., 13, 102-108. Warden, C.H., Yi, N. and Fisler, J. (2004) Epistasis among genes is a universal phenomenon in obesity: evidence from rodent models. Nutrition, 20, 74-77. Lewis, C.E., North, K.E., Arnett, D., Borecki, I.B., Coon, H., Ellison, R.C., Hunt, S.C., Oberman, A., Rich, S.S. and Province, M.A.(2005) Sex-specific findings from a genome-wide linkage analysis of human fatness in non-Hispanic whites and African Americans: the HyperGEN study. Int. J. Obes. Relat. Metab. Disord., 29, 639-649).

There have been many attempts to identify genes involved in the predisposition to common obesity, but presumably because of the genetic complexity, these efforts have had only mixed success (Bell, C.G., Walley, A.J. and Froguel, P. (2005) The genetics of human obesity. Nat. Rev. Genet., 6, 221-234., Pérusse, L., Rankinen, T., Zuberi, A., Chagnon, Y.C., Weisnagel, S.J., Argyropoulos, G., Walts, B., Snyder, E.E. and Bouchard, C. (2005) The human obesity gene map: the 2004 update. Obes. Res., 13, 381-490). Studies focused on rare forms of obesity have been more productive. Mutations in several genes have been linked to monogenic obesity. Also, several genes have been implicated in syndromic obesity (i.e. obesity with other clinically significant phenotypes not usually associated with the disease). However, genetic causes of the common form of this disease remain poorly defined.

Dietary fat intake is considered a major factor in the development of obesity and insulin resistance. In mice, sensitivity to dietary fat is strain specific, and consequently dependent on a genetic basis (West, D.B., Boozer, C.N., Moody, D.L. and Atkinson, R.L. Dietary obesity in nine inbred mouse strains. Am J Physiol 262, R1025-32 (1992). Wuschke, S., Dahm, S., Schmidt, C., Joost, H.G. and Al-Hasani, H. A meta-analysis of quantitative trait loci associated with body weight and adiposity in mice. Int J Obes Lond) (2006) Rankinen, T. et al. The human obesity gene map: the 2005 update. Obesity (Silver Spring) 14, 529-644 (2006)). Weight gain in response to dietary fat/caloric intake is dependent on the genetic composition of an individual. However, the genes and variants that determine the sensitivity to dietary fat are largely unknown. Since obesity is a major risk factor for the development of type-2 diabetes mellitus, cardiovascular diseases, and perhaps certain types of cancer, it is imperative to identify genetic risk factors for diet-induced adiposity and weight gain. Moreover, it is well established that obesity coincides with reduced insulin sensitivity and promotes impaired glucose tolerance. Nevertheless, it is still debated how increased fat mass affects insulin action on the molecular level.

The sequence of *Homo sapiens* TBC1 (tre-2/USP6, BUB2, cdc16) domain family, member 1 (TBC1D1) is described in the database under Accession number NM_015173. TBC1D1 is described as a founding member of a family of proteins sharing a 180- to 200-amino acid TBC domain presumed to have a role in regulating cell growth and differentiation. These proteins share significant homology with TRE2 (USP6; MIM 604334), yeast Bub2, and CDC 16 (MIM 603461)

WO 00/08209 concerns genomic and cDNA sequences of the human TBC-1 gene and polypeptides encoded by the TBC-1 gene. WO 00/08209 also deals with antibodies directed specifically against such polypeptides that are useful as diagnostic reagents in *prostate cancer.* WO 00/08209 further encompasses biallelic markers of the TBC-1 gene useful in genetic analysis.

Stone et al. (in Stone S, Abkevich V, Russell DL, Riley R, Timms K, Tran T, Trem D, Frank D, Jammulapati S, Neff CD, Iliev D, Gress R, He G, Frech GC, Adams TD, Skolnick MH, Lanchbury JS, Gutin A, Hunt SC, Shattuck D. TBC1D1 is a candidate for a severe obesity gene and evidence for a gene/gene interaction in obesity predisposition. Hum Mol Genet. 2006 Sep 15; 15(18):2709-20. Epub 2006 Aug 7) describe a genetic variation in the human gene TBC1D1 that confers risk for severe obesity in females. They identified a coding variant (R125W) in TBC1D1 that segregated with the disease in 4p15-14-linked obesity pedigrees. They postulate that R125W affects obesity susceptibility, delimits the location of an obesity gene at 4q34-35, and mention that the protein is structurally similar to a known regulator of insulin-mediated Glut4 translocation (AS 160).

Stone et al. further describe that a) R125W confers risk for obesity only in the context of a specific gene/gene interaction, b) the W allele is not enriched in random severely obese cases, and c) that TBC1D1 confers risk for severe obesity in females. Stone et al. do not describe d) deletions or truncations of TBC1D1, e) a lack of functionality of TBC1D1 in obese cases, and f) a link of TBC1D1 with the diet of the obese cases.

In view of the above, it is an object of the present invention, to provide novel genetic markers that are involved in the response to dietary fat, and obesity. These markers provide valuable tools both for diagnostic as well as therapeutic approaches in order to treat or prevent obesity.

According to a first aspect thereof, the object of the present invention is solved by providing a method for diagnosing obesity or diabetes or an increased risk for obesity or diabetes in a mammal, comprising analysis of the biological activity and/or expression of TBC1D1 in a biological sample obtained from said mammal to be diagnosed, wherein a lack or reduced expression of the gene for TBC1D1 or a lack or reduced biological activity of TBC1D1 in said mammal, when compared to a biological sample obtained from a non-diseased mammal, is indicative for an absence or a reduced risk for obesity or diabetes in said mammal.

According to another aspect thereof, the object of the present invention is solved by providing a screening tool for an agent for treating or preventing obesity or diabetes, wherein said tool is selected from a) a polynucleotide according to SEQ ID NO: 3 or 4 (providing the human and mouse mRNA sequences, respectively), b) a polypeptide that is encoded by SEQ ID NO: 3 or 4, in particular having an amino acid sequence of SEQ ID NO: 1 or 2 (providing the human and mouse amino acid sequences, respectively), c) a polypeptide having an amino acid sequence having a 90% or more homology with an amino acid sequence of SEQ ID NO: 1 or 2, and exhibiting a TBC1D1 activity, and d) a polypeptide having an amino acid sequence in which 1 to 10 amino acids are deleted, substituted, and/or added in the amino acid sequence according to SEQ ID NO: 1 or 2, and exhibiting a TBC1D1 activity.

According to yet another aspect thereof, the object of the present invention is solved by providing a method of identifying a compound that modulate the expression and/or biological activity of TBC1D1 in a cell, comprising the steps of a) contacting a cell expressing TBC1D1 with at least one potentially interacting compound, and b) measuring the modulation of the expression and/or biological activity of TBC1D1 in said cell. Preferred is a method according to the present invention, wherein said compound as identified is an inhibitor of the expression and/or biological activity of TBC1D1 in said cell.

Another aspect of the present invention then relates to a pharmaceutical composition for treating or preventing obesity or diabetes, which includes a compound that modulate the expression and/or biological activity ofTBC1D1 in a cell obtainable by a method according to the present invention.

Another aspect of the present invention then relates to the use of a modulator of the expression and/or the biological activity of TBC1D1 in a cell for the manufacture of a pharmaceutical composition for treating or preventing obesity or diabetes. Preferred is a use according to the present invention, wherein said obesity or diabetes is dietary fat-related. Further preferred is a use according to the present invention, wherein said modulator is an inhibitor of the expression and/or biological activity of TBC1D1.

The present invention is technically based on genome wide scans of outcross populations of the New Zealand Obese (NZO) mouse and the lean Swiss Jim Lambert (SJL) strain, whereby the inventors have previously identified a major QTL for high fat diet (HFD) induced obesity *(Nob1;* LOD score 7.9) on chromosome 5. Additional crossbreeding experiments indicated that *Nob1* represents an obesity suppressor from the SJL strain.

In a combined approach of gene expression analysis and sequencing, the inventors identified a SJL specific 7 bp deletion in the *Tbc1d1* gene (4048delACTCGCT) which results in a truncated protein lacking the TBC/Rab GTPase activating (GAP) domain. Tbc1d1 was identified by genetic and molecular analyses of mouse models that are inherently sensitive or resistant to diet-induced obesity and diabetes, respectively. The data of the inventors indicated that the integrity of the Tbc1d1 gene product is required for diet-induced weight gain. In fact, Tbc1d1 activity (presumably through its GTPase activating protein (GAP) domain) appears to determine the capacity of gaining weight in response to a given caloric input: According to the data, animals with a high activity/high amount of intact Tbc1d1 gene product gain more weight than animals with low activity/low amount of intact Tbc1d1 in response to a caloric challenge, such as high fat diet induced obesity. This phenomenon is designated herein as "dietary fat-related", although the caloric challenge is not exclusively limited to calories derived from dietary fat.

Tbcld1 is a member of a larger family of putative signalling proteins that are highly conserved in mammals. Mouse and human Tbc1d1 proteins are 80-90% identical, and the tissue expression profile of Tbc1d1 appears to be identical in both species. Tbc1d4 also known as AS160 is a critical protein for insulin signalling, and highly homologous to Tbc1d1. It is established that Tbcld4 exerts the same crucial function in insulin signalling in both species, human and mouse. Thus, the biological function of Tbc1d1 is highly likely to be conserved between species. As a matter of fact, a previous study from the Utah group (Stone et al., see above) has reported genetic evidence of linkage of the Tbc1d1 locus to obesity in humans.

Introgression of the *Nob1* segment of the SJL chromosome 5 into a mixed NZO/C57BL/6J background markedly reduces body weight and blood glucose levels. Tbc1d1 is related with the insulin signalling protein Tbc1d4 (AS 160), and is highly expressed in skeletal muscle, heart, pancreas, kidney and hypothalamus; its expression in skeletal muscle is increased by high fat diet. The data strongly suggest that deletion of *Tbc1d1* suppresses high fat diet induced obesity caused by the polygenic NZO background. Thus, *Tbc1d1* appears to be involved in a novel pathway that regulates lipid metabolism and energy homeostasis in skeletal muscle.

In view of this, possible applications of the present invention for humans include:
a) Diagnostic approaches: Since Tbc1d1 activity determines the susceptibility for (high fat) diet-induced weight gain, genetic tests can be developed to assess an individual risk for developing overweight/obesity and or diabetes, and to develop a personalized diet and/or treatment as an intervention approach (see above). These tests are also envisaged to be applicable for diagnosing/treating weight disorders in general, i.e. also weight loss as a result of e.g. eating disorders or during cancer therapy.
b) Pharmaceutical (obesity/diabetes) and therapeutic approaches: The inventors' data indicate that an inhibitor of Tbc1d1 action can be used as an obesity drug in mammals/humans. Moreover, since reduced levels of Tbc1d1 also result in reduced blood glucose levels in a mouse model for the metabolic syndrome, a Tbc1d1 inhibitor is very likely to be used as a diabetes drug as well, and also for treating other components of the metabolic syndrome, including hyperlipidemia, hypercholesterinemia, high blood pressure, and aspects of vascular and cardiovascular diseases that are usually secondary to insulin resistance and diabetes. Since the lean SJL mice that are deficient in Tbc1d1 appear to be healthy, normoglycemic and without major metabolic derangements, a pharmacological inhibitor of Tbc1d1 is likely to have a high therapeutic index, i.e. appears to be tolerable and safe.

Furthermore, as mentioned above, high fat diet is known to induce insulin resistance, obesity and diabetes in certain individuals. Interestingly, the majority of individuals with type-2 diabetes are obese. However, only a minor fraction of obese individuals develop type-2 diabetes. Thus, certain variants of Tbcld1 that might interact with the environment (e.g. dietary fat) could provide a link between diabetes and obesity. Tbc1d1 is highly expressed in skeletal muscle, the main site of glucose disposal and an important mediator or energy homeostasis. The inventors' data show that Tbc1d1 is phosphorylated at a specific threonine by AKT2 kinase in response to insulin. Currently, a possible relationship between impaired insulin action in skeletal muscle in the insulin resistant state and obesity is investigated further.

Moreover, tbc1d1 appears to define a novel pathway with potentially valuable downstream targets that regulate lipid metabolism, energy homeostasis and/or other, yet unknown biological entities.

Finally, the ortholog of the marker tbc1d1 could be of use in order to identify animals that gain more weight in response to a caloric, such as a high fat-containing, diet. The marker thus could be used for breeding purposes of cattle by, for example, selecting those animals that do not have a mutated tbc1d1 marker.

The term "homology" as used herein means a value obtained by a BLAST [Basic local alignment search tool; Altschul, S. F. et al., J. Mol. Biol., 215, 403-410, (1990)] search. The homology in the amino acid sequence may be calculated by a BLAST search algorithm. More particularly, it may be calculated using a b12seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap insertion cost value, "0" as a Gap elongation cost value, "SEG" as a filter for a Query sequence, and "BLOSUM62" as a Matrix are used, respectively.

Preferred is a diagnostic or therapeutic method according to the present invention, wherein said obesity or diabetes is dietary fat-related. As a major finding in the context of the present invention, animals with a high activity/high amount of intact Tbc1d1 gene product gain more weight than animals with low activity/low amount of intact Tbc1d1 in response to a caloric challenge, such as high fat diet. Thus, the animals with a high activity/high amount of intact Tbc1d1 gene product do not always become obese, but gain more weight in response to a caloric challenge, such as a high fat-containing diet.

Preferred is a diagnostic or therapeutic method according to the present invention, wherein said mammal to be diagnosed or treated is a mouse, rat or human.

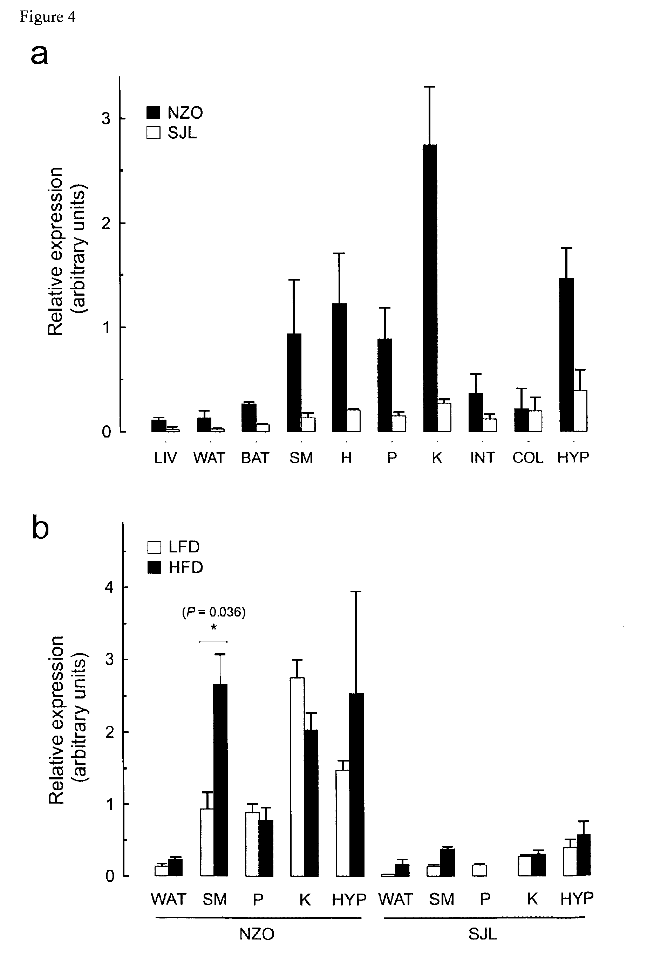

Preferred is a diagnostic or therapeutic method according to the present invention, wherein said biological sample contains biological material that is derived from skeletal muscle, heart, pancreas, kidney, and/or hypothalamus of said mammal to be diagnosed. In C57BL/6J and NZO mice, *Tbc1d1* is expressed mainly in skeletal muscle, heart, pancreas, kidney and hypothalamus, and only low levels of expression were detected in white and brown adipose tissue, liver, intestine and colon (see Figure 4a).

Both the expression and/or abundance of TBC1D1 or the mRNA thereof as well as the biological activity can be determined with any suitable assay known to the person of skill. Common assays involve PCR, Northern-, Western- or Southern-Blots, and/or enzymatic assays. Thus, preferred is a method according to the present invention, wherein said diagnosis comprises the use of TBC1D1-specific antibodies, hybridisation analysis, quantitative PCR, mRNA analysis, quantitative analysis of the amount of TBC1D1-protein, sequencing of the TBC1D1-locus, and/or an analysis of the activity of TBC1D1 in said sample.

Further preferred is a method according to the present invention, wherein TBC1D1 is down-regulated at least 3-fold in skeletal muscle. As found during the experiments in the context of the present invention, in skeletal muscle of C57BL/6J and NZO mice, expression of *Tbc1d1* is significantly increased ~3-fold in response to high fat diet, whereas no significant changes were observed in the other tissues (Figure 4b). As a result, in skeletal muscle from animals raised on a high-fat diet, the levels of *Tbc1d1* mRNA are increased by about an order of magnitude in NZO mice compared to SJL mice, respectively.

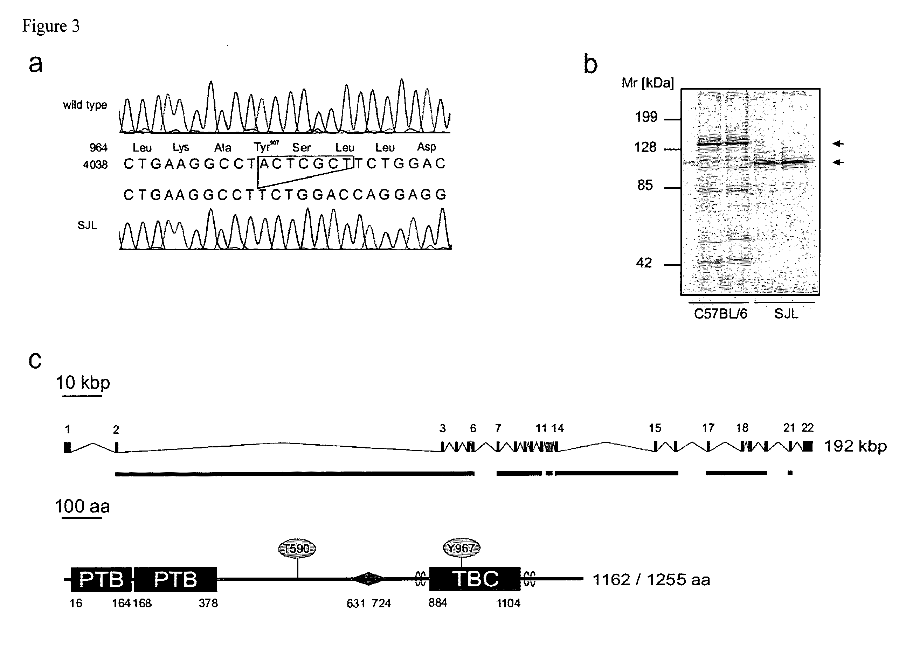

In a particular preferred embodiment of the method according to the present invention, the gene for TBC1D1 contains a deletion, in particular loss-of-function deletion, more particularly a 7 bp deletion in exon 18 (A⁴⁰⁴⁸ - T⁴⁰⁵⁴ Genbank Acc No. AK122445) of the mouse gene for TBC1D1 or the homologous deletion in the human gene for TBC1D1. As identified in the in vitro translation studies in the context of the present invention (Figure 3), the deletion represents a loss-of function (LOF) mutation since the mutated protein lacks most invariant residues of the GTPase-activating TBC domain, including the conserved and catalytically essential glutamine finger (YxQ978) (Pan, X., Eathiraj, S., Munson, M. and Lambright, D.G. TBC-domain GAPs for Rab GTPases accelerate GTP hydrolysis by a dual-finger mechanism. Nature 442, 303-6 (2006)). In a particular preferred embodiment of the method according to the present invention, the biological activity of TBC1D1 is the activity of the Rab-specific GTP-ase activating TBC domain.

As already mentioned above, according to another aspect thereof, the object of the present invention is solved by providing a screening tool for an agent for treating or preventing obesity or diabetes, wherein said tool is preferably selected from a polypeptide consisting of an amino acid sequence of SEQ ID NO: 1 or 2 or an mammalian ortholog of these polypeptides (by definition, orthologs are genes that are related by vertical descent from a common ancestor and encode proteins with the same function in different species).

In another aspect of the present invention, the screening tool for an agent for treating or preventing obesity or diabetes is a cell which is transformed with an expression vector comprising a polynucleotide encoding a polypeptide according to SEQ ID NO: 1 or 2 or an mammalian ortholog of these polypeptides, and expresses said polypeptide. The cell can be a prokaryotic or eukaryotic cell, and the expression constructs can be present extrachromosomally or integrated into the chromosome. The polypeptide can be expressed in the form of a fusion protein, for example together with an enzymatically active moiety as reporter-construct, in order to be able to detect the expression product. Preferred host cells are derived from cells selected from the skeletal muscle, heart, pancreas, kidney, and/or hypothalamus.

In another aspect of the present invention, the screening tool for an agent for treating or preventing obesity or diabetes is a non-human transgenic mammal overexpressing TBC1D1 and/or carrying a TBC1D1 genetic reporter-construct. Preferred is a transgenic mouse, rat, pig, goat or sheep, wherein the reporter-construct is preferably expressed in cells selected from the skeletal muscle, heart, pancreas, kidney, and/or hypothalamus of said animal. Methods to produce these non-human transgenic mammal overexpressing TBC1D1 and/or carrying a TBC1D1 genetic reporter-construct are well known to the person of skill in the art.

As already mentioned above, according to yet another aspect thereof, the object of the present invention is solved by providing a method of identifying a compound that modulate the expression and/or biological activity of TBC1D1 in a cell, comprising the steps of a) contacting a cell expressing TBC1D1 with at least one potentially interacting compound, and b) measuring the modulation of the expression and/or biological activity of TBC1D1 in said cell. Preferred is a method according to the present invention, wherein said compound as identified is an inhibitor of the expression and/or biological activity of TBC1D1 in said cell.

This method is suitable for the determination of compounds that can interact with TBC1D1 and to identify, for example, inhibitors, activators, competitors or modulators of TBC1D1, in particular inhibitors, activators, competitors or modulators of the enzymatic activity of TBC1D1. Another aspect is directed at compounds that modulate the expression of TBC1D in a cell/in cells.

The term "contacting" in the present invention means any interaction between the potentially binding substance(s) with TBC1D1, whereby any of the two components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip and TBC1D1 (or a functional part thereof) is subsequently contacted with such a chip.

The TBC1D1 employed in a method of the present invention can be a full length protein or a fragment with N/C-terminal and/or internal deletions. Preferably the fragment is either an N-terminal fragment comprising the enzymatic region of the polypeptide or a C-terminal fragment comprising the cytoplasmic region, depending on whether potentially interacting compounds are sought that specifically interact with the N- or C-terminal fragment.

The potentially binding substance, whose binding to TBC1D1 is to be measured, can be any chemical substance or any mixture thereof. For example, it can be a substance of a peptide library, a combinatory library, a cell extract, in particular a plant cell extract, a "small molecular drug", a protein and/or a protein fragment.

Measuring of binding of the compound to TBC1D1 can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of either TBC1D1 or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to TBC1D1 or TBC1D1 fragments. The effect of the binding of the compound or the activity of TBC1D1 can also be measured indirectly, for example, by assaying an enzymatic activity of TBC1D1 after binding, such as the phosphorylation at a specific threonine by AKT2 kinase in response to insulin, and the like.

As a further step after measuring the binding of a potentially interacting compound and after having measured at least two different potentially interacting compounds at least one compound can be selected, for example, on grounds of the measured binding activity or on grounds of the detected increase or decrease of TBC1D1 activity and/or expression.

The thus selected binding compound is then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The thus modified binding substances are than individually tested with a method of the present invention, i.e. they are contacted with TBC1D1 and subsequently binding of the modified compounds to the TBC1D1 polypeptide is measured. In this step, both the binding per se can be measured and/or the effect of the function of the TBC1D1 like, e.g. the enzymatic activity of the polypeptide can be measured. If needed the steps of selecting the binding compound, modifying the binding compound, contacting the binding compound with a TBC1D1 polypeptide and measuring the binding of the modified compounds to the protein can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity of the TBC1D1 polypeptide(s).

In a further embodiment of the method of the present invention, the interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routs of application like, for example, intravenous solution, sprays, band-aids or pills.

Similar to the strategies for identifying compounds that interact with TBC1D1, compounds can be identified that modulate the expression of TBC1D1 in a cell. In preferred strategies, the expression of TBC1D1 1 can be monitored using a reporter-construct for TBC1D1 (in order to analyse the translation efficiency and/or stability of the TBC1D1 polypeptide), for an example a fusion protein comprising a detectable fusion member (such as an enzymatic or fluorophoric group), or the amount of mRNA as present in a cell can be measured, for example, by Northern blot. The expression can also be analysed and monitored by using chip-analysis or rtPCR. Preferred compounds that modulate the expression of TBC1D1 in a cell are selected from specific antisense oligonucleotide(s), or siRNA(s) for RNAi. Respective methods are well described in the literature and known to the person of skill.

Another aspect of the present invention relates to a method for manufacturing a pharmaceutical composition for treating or preventing obesity or diabetes, comprising the steps of: performing a screening method according to the present invention, and formulating said modulator as screened and identified into a pharmaceutical composition.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

Administration of an agent, e.g., a compound can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or nonsolvent. In many cases, water or an organic liquid can be used.

Yet another aspect of the present invention is directed at a pharmaceutical composition for treating or preventing obesity or diabetes, obtainable by a method according to the method as above.

In certain embodiments, the compound (modulator) is administered to the subject by administering a recombinant nucleic acid. Preferably, the recombinant nucleic acid is a gene therapy vector.

Another aspect of the present invention relates to a method or use as above, wherein the pharmaceutical composition further comprises additional pharmaceutically active ingredients that modulate the diseases to be treated, such as obesity and diabetes.

Another aspect of the present invention relates to a method for treating or preventing obesity or diabetes, comprising a diagnostic method according to the present invention as above, and providing a treatment to said mammal based on, at least in part, the result of said diagnosis. In general, the attending physician will base a treatment, and in particular obesity- and/or diabetes-preventive measures, on the diagnostic data regarding TBC1D1 of said patient, wherein said patient diagnostic data regarding TBC1D1 can be integrated with other individual patient data (clinical data, family history, DNA, etc.), and a treatment can also be performed based on the combination of these factors. This method of the present invention for example involves integrating individual diagnostic data with patient clinical information and general healthcare statistics to enable, for example, the application of personalized medicine to the mammal. Significant information about drug effectiveness, drug interactions, physiologic heart regularities and other patient status conditions could be correlated with the specific diagnostic data.

A further aspect of the present invention is then a method of treating or preventing obesity or diabetes in a mammal, comprising administering to said mammal an effective amount of a pharmaceutical composition according to the invention as above. Preferably, an inhibiting active agent is administered in form of a pharmaceutical composition, such as an antibody, antisense nucleotide or an inhibiting binding compound. Preferably, said patient is a human being. Treating is meant to include, e.g., preventing, treating, reducing the symptoms of, or curing the disease or condition.

Another aspect then relates to a monoclonal antibody recognizing an epitope derived from the N-terminus of mouse Tbc1d1 (Genbank NP_062610) amino acids 215-229: CTGQPSAPGPRPMRKS-CONH2 (Cys1 is used for coupling) or an ortholog of this peptide sequence.

An "effective amount" is an amount of the compound(s) as mentioned above that a) acts on the expression and/or abundance of TBC1D1 as analysed, and which alleviates symptoms as found for obesity (which might overlap with diabetic symptoms in general). Alleviating is meant to include, e.g., preventing, treating, reducing the symptoms of, or curing the disease or condition.

The invention also includes a method for treating a subject at risk for obesity and/or diabetes, in particular dietary fat-related, wherein a therapeutically effective amount of a modulator as above is provided. Being at risk for the disease can result from, e.g., a family history of the disease, a genotype which predisposes to the disease, or phenotypic symptoms which predispose to the disease. A further aspect of the present invention is the use of a modulator of the expression and/or the biological activity of TBC1D1 in a cell for the manufacture of a pharmaceutical composition for treating or preventing obesity or diabetes. Preferred is a use, wherein said obesity or diabetes is dietary fat-related (as described above). More preferably, said modulator is an inhibitor of the expression and/or biological activity of TBC 1 D 1.

The following figures, sequences, and examples merely serve to illustrate the invention and should not be construed to restrict the scope of the invention to the particular embodiments of the invention described in the examples. All references cited in the text are hereby incorporated in their entirety by reference.

SEQ ID NOs 1 and 2 show the polypeptides TBC1D1 that are encoded by SEQ ID NO: 3 or 4, respectively.

SEQ ID NOs 3 and 4 show the nucleotide sequence of the mouse gene for TBC1D1 (Genbank Acc No. AK122445) and the human gene for TBC1D1 (Genbank Acc No. NM_015173), respectively.

SEQ ID NOs 5 to 102 show primers and probes as used in the examples below (see also table 1).

### Figures

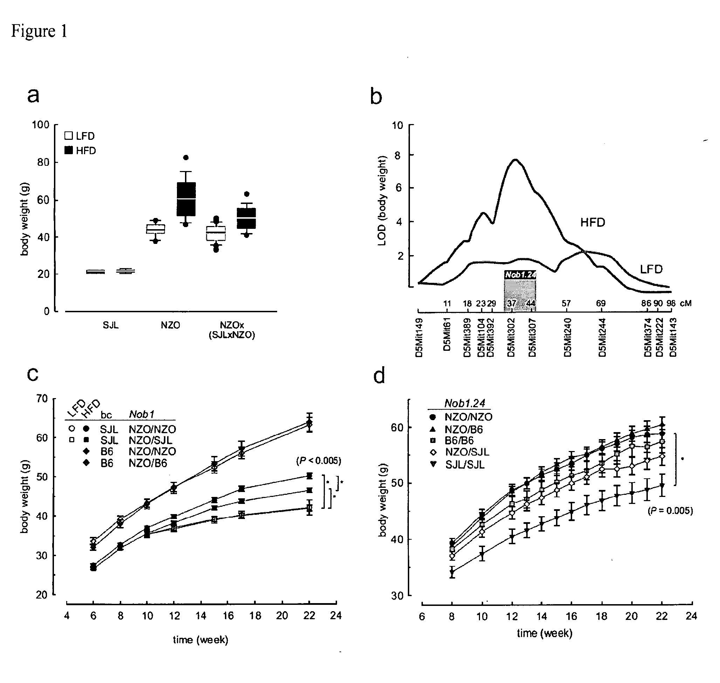

**Figure 1** shows that the adiposity QTL *Nob1* constitutes a diet-dependent obesity suppressor from the lean SJL strain. (a) boxplot representation of the body weight distribution at week 22 of SJL mice, NZO mice, and a NZO × F1(SJL×NZO) backcross population raised on a low-fat (LFD) or high-fat diet (HFD), respectively. (b) LOD ratios on chromosome 5 for body weight at week 22 in the combined NZO × F1(SJL×NZO) backcross population on a LFD (n= 297) or HFD (n= 585). Body weights were adjusted for gender differences. *Nob1.24* represents the critical 24 Mb region of the *Nob1*-QTL. (**c**) *Nob1* genotype- and diet-dependent body weight (± s.e.m) of female mice from two different backcross populations of NZO and SJL (SJL bc; n= 17 - 89), and NZO and C57BL/6J (B6 bc; n= 25-34). (**d**) genotype-dependent body weight (± s.e.m) of female F2 mice from a cross of RCS B6.SJL-*Nob1.24* and NZO raised on a HFD (n= 19 - 58).

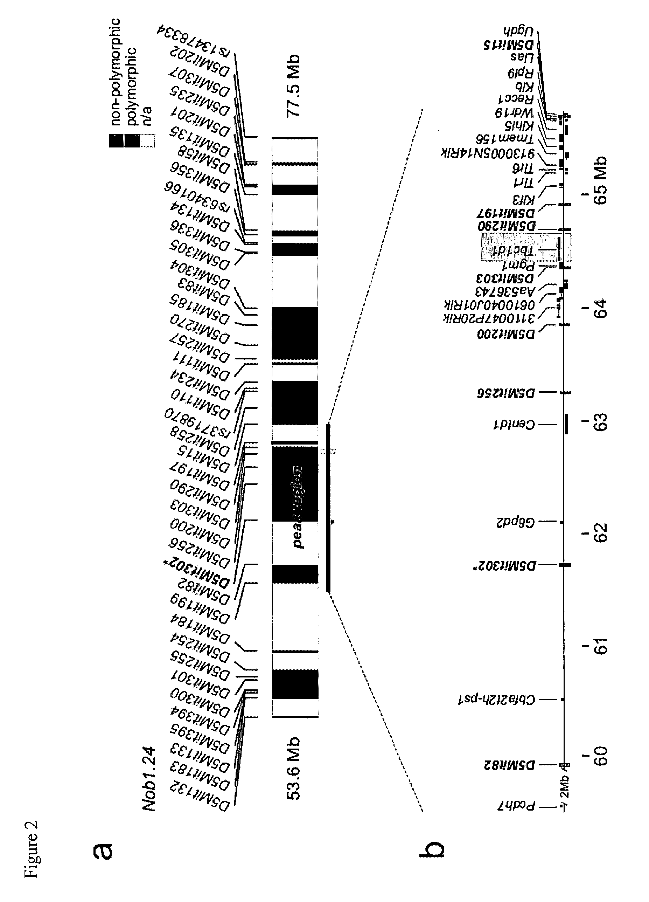

**Figure 2** shows fine mapping of the critical region of *Nob1.* (**a**) The chromosomal segment from SJL in RCS B6.SJL-*Nob1.24* mice was used for pairwise comparison of microsatellite fragment length polymorphisms and SNPs between parental NZO and SJL mice. The markers define polymorphic (red) and non-polymorphic regions (green), and regions residing between adjacent blocks typed as NZO or SJL (white). (**b**) Physical gene map of the *Nob1* peak region.

**Figure 3** shows a SJL-specific deletion in exon 18 of *Tbc1d1* which results in premature termination of the protein and deletion of the TBC domain. (**a**) Electropherograms of the wild type (C57BL/6J) and mutant (SJL) alleles of *Tbc1d1* showing sequences around the sites of the deletion (del(A4048-T4054)). The frameshift mutation results in termination of the protein at TGA⁴¹²⁹. (**b**) Autoradiograph of *in vitro* translated [³⁵S]*Tbc1d1* (arrows) from C57BL/6J (app. Mᵣ ~141 kDa) and SJL (app. Mᵣ ~117 kDa) after 10% SDS-PAGE. (**c**) Gene structure of *Tbc1d1* and proposed domain architecture of the protein. Indicated is the conserved phosphorylation site for AKT (T⁵⁹⁰) and site of the mutation (Y⁹⁶⁷) in the protein. PTB, phosphotyrosine binding domain; TBC, tre-2/USP6, BUB2, cdc 16 domain; CC, coiled-coil domain; blue diamond, 93 amino acid insertion by alternative splicing of exons 12 and 13.

**Figure 4** shows the tissue distribution and diet-induced expression of *Tbc1d1.* (**a**) Relative expression levels of *Tbc1d1* in different tissues. (**b**) Expression levels in tissues from LFD and HFD fed mice. Quantification of gene expression was performed with samples from 8 wk old mice (n = 6) by TaqMan-Realtime-PCR analysis and normalization to the expression of β-actin. Values represent means ± s.d. LIV, liver; WAT, white adipose tissue; BAT, brown adipose tissue; SM, skeletal muscle; H, heart; P, pancreas; K, kidney; INT, intestine; COL; colon; HYP, hypothalamus.

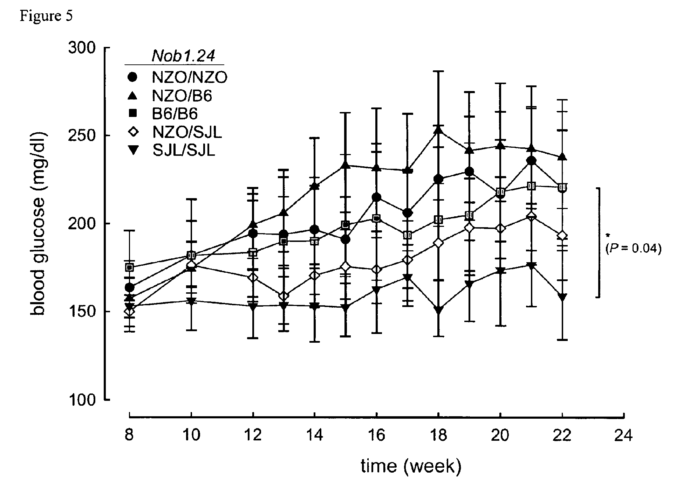

**Figure 5** shows the genotype-dependent blood glucose levels (± s.e.m) of female F2 mice from a reporter cross of RCS B6.SJL-*Nob1.24* and NZO raised on a HFD (n= 19 - 58). Blood glucose levels are represented as re-transformed geometric means with 95% confidence interval.

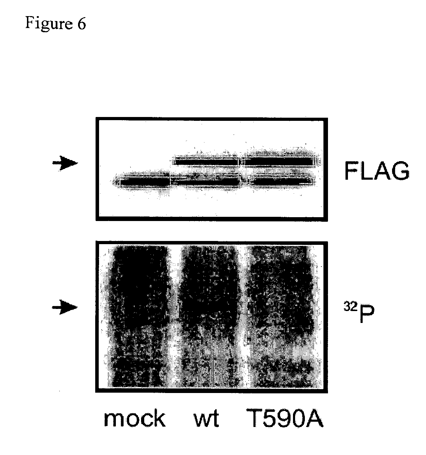

**Figure 6** shows the phosphorylation of T⁵⁹⁰ in *Tbc1d1* by AKT2 kinase *in vitro.* FLAG-tagged wild type *Tbc1d1* and T590A mutant was immunoprecipitated and incubated with purified recombinant AKT2 kinase (~1 µM) in presence of 100 µM [γ³²-P]ATP for 15 min as described in "Methods". An equal amount of the immunoprecipitate was blotted for the FLAG tag. Phosphate incorporation was visualized by autoradiography. Arrows denote the position of *Tbc1d1.*

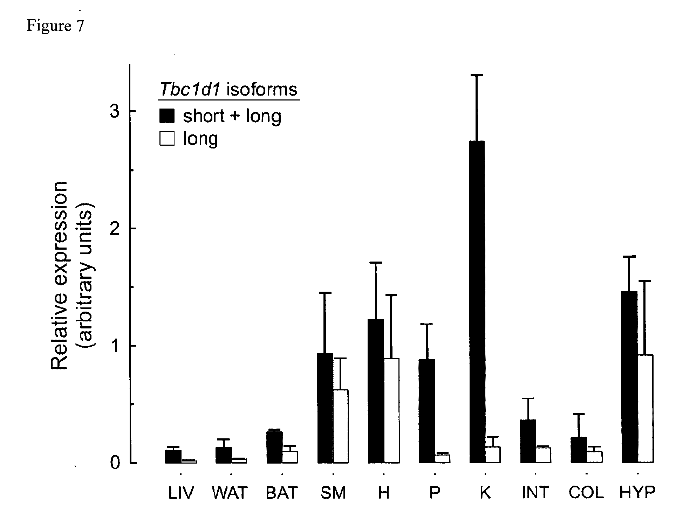

**Figure 7** shows the tissue distribution of the two *Tbc1d1* isoforms. Quantification of gene expression was performed with samples from 8 wk old mice (means ± s.d.; n = 5-6) by TaqMan-Realtime-PCR analysis and normalization to the expression of β-actin. LIV, Liver; WAT, white adipose tissue; BAT, brown adipose tissue; SM, skeletal muscle; H, heart; P, pancreas; K, kidney; INT, intestine; COL; colon; HYP, hypothalamus.

**Table 1** shows the primer and probe sequences.

### Examples

In order to identify dietary fat responsive genetic loci in mice as a model for human obesity, the inventors performed crossbreeding experiments of the New Zealand Obese (NZO) mouse and the lean Swiss Jim Lambert (SJL) strain. The NZO strain presents a metabolic syndrome with early onset polygenic obesity, dyslipidemia, hypertension and a type-2 like diabetes mellitus (Crofford, O.B. and Davis, C.K., Jr. Growth Characteristics, Glucose Tolerance and Insulin Sensitivity of New Zealand Obese Mice. Metabolism 14, 271-80 (1965). Leiter, E.H. et al. NIDDM genes in mice: deleterious synergism by both parental genomes contributes to diabetogenic thresholds. Diabetes 47, 1287-95 (1998). Ortlepp, J.R. et al. A metabolic syndrome of hypertension, hyperinsulinaemia and hypercholesterolaemia in the New Zealand obese mouse. Eur J Clin Invest 30, 195-202 (2000). Jurgens, H.S. et al. Hyperphagia, lower body temperature, and reduced running wheel activity precede development of morbid obesity in New Zealand obese mice. Physiol Genomics 25, 234-41 (2006).) Moreover, NZO mice are highly susceptible to weight gain when fed a high-fat diet (HFD), resulting in the development of morbid obesity with fat depots exceeding 40% of total body weight (Jurgens, H.S. et al. Hyperphagia, lower body temperature, and reduced running wheel activity precede development of morbid obesity in New Zealand obese mice. Physiol Genomics 25, 234-41 (2006). Giesen, K., Plum, L., Kluge, R., Ortlepp, J. and Joost, H.G. Diet-dependent obesity and hypercholesterolemia in the New Zealand obese mouse: identification of a quantitative trait locus for elevated serum cholesterol on the distal mouse chromosome 5. Biochem Biophys Res Commun 304, 812-7 (2003). Jurgens, H.S. et al. Development of diabetes in obese, insulin resistant mice: Essential role of dietary carbohydrates in beta-cell destruction. Diabetologia (in press)(2007)). In contrast, the lean SJL strain is resistant to HFD-induced obesity and diabetes (West, D.B., Boozer, C.N., Moody, D.L. and Atkinson, R.L. Dietary obesity in nine inbred mouse strains. Am J Physiol 262, R1025-32 (1992). Giesen, K., Plum, L., Kluge, R., Ortlepp, J. and Joost, H.G. Diet-dependent obesity and hypercholesterolemia in the New Zealand obese mouse: identification of a quantitative trait locus for elevated serum cholesterol on the distal mouse chromosome 5. Biochem Biophys Res Commun 304, 812-7 (2003)) (Figure 1a). The propensity of NZO mice for HFD-induced body weight gain was retained in the NZO x F1 (SJL x NZO) backcross population (Figure 1a). However, whereas backcross mice and parental NZO animals had similar mean body weights on a low fat diet (LFD), the HFD-induced weight gain of the backcross population was diminished (Figure 1a). In a subsequent genome-wide scan of the backcross population, we identified *Nob1,* a major quantitative trait locus (QTL) on chromosome 5 for high-fat diet (HFD)-induced obesity (body weight, body mass index, total body fat; LOD score 7.9; Figure 1b) (Kluge, R. et al. Quantitative trait loci for obesity and insulin resistance (Nob1, Nob2) and their interaction with the leptin receptor allele (LeprA720T/T1044I) in New Zealand obese mice. Diabetologia 43, 1565-72 (2000). Plum, L. et al. Type 2 diabetes-like hyperglycemia in a backcross model of NZO and SJL mice: characterization of a susceptibility locus on chromosome 4 and its relation with obesity. Diabetes 49, 1590-6 (2000)). So far, three further adipogenic alleles from NZO have been mapped to chromosomal regions proximal *(Obq11*, 23 Mb; *Obq12,* 51 Mb) and dista113 (D5Mit7, 93 Mb) of *Nob1,* indicating the presence of additional NZO derived adiposity loci on this chromosome. In addition to its interaction with dietary fat8, *Nob1* showed strong epistatic interaction with *Nidd*/*SJL,* a QTL for pancreatic islet cell destruction 11: carriers of the NZO allele of *Nob1* exhibited a pronounced acceleration of the development of diabetes when fed a high fat diet (Giesen, K., Plum, L., Kluge, R., Ortlepp, J. and Joost, H.G. Diet-dependent obesity and hypercholesterolemia in the New Zealand obese mouse: identification of a quantitative trait locus for elevated serum cholesterol on the distal mouse chromosome 5. Biochem Biophys Res Commun 304, 812-7 (2003). Plum, L. et al. Characterisation of the mouse diabetes susceptibilty locus Nidd/SJL: islet cell destruction, interaction with the obesity QTL Nob1, and effect of dietary fat. Diabetologia 45, 823-30 (2002)).

In the NZO/SJL backcross population, heterozygous carriers of the SJL allele at *Nob1* and the corresponding homozygous carriers of the NZO allele were not different in body weight when fed a LFD (Figure 1c). In contrast, *Nob1*NZO/NZO animals showed a markedly increased body weight on a HFD compared with the heterozygous *Nob1*NZO/SJL animals, indicating that the diet-induced weight gain is either increased by the NZO allele or decreased by the SJL allele of *Nob1.* To assess the net effect of the NZO allele of *Nob1,* we conducted additional crossbreeding experiments with NZO and C57BL/6J laboratory mice. However, in the corresponding NZO/C57BL/6J backcross there was no correlation of the *Nob1* genotype and HFD induced body weight gain, and no linkage of diet-induced body weight to chromosome 5, indicating that *Nob1* may represent an obesity-suppressor gene from the lean SJL stain (Figure 1c). To validate the obesity-suppressive effect of *Nob1SJL* on a different genetic background and to narrow down the critical region of the Nobl-QTL, we generated a recombinant congenic mouse strain (B6.SJL-*Nob1.24*) harbouring a 24 Mb fragment (53.6Mb - 77.5 Mb) of the *Nob1* region from SJL on a C57BL/6J background (Figure 1b).

Introgression of the *Nob1*.24 segment from SJL into a mixed NZO/C57BL/6J background replicated the lean phenotype of SJL (Figure 1d). On a high fat diet, both homozygous and heterozygous carriers of the *Nob1*SJL allele show markedly reduced body weight and blood glucose levels compared to control animals, respectively (Figure Id and Figure 5). Hence, *Nob1* represents a suppressor for HFD-induced obesity, associated with a 24 Mb segment on chromosome 5 from the lean SJL strain.

For positional cloning of the *Nob1* gene we performed fine mapping of the *Nob1.24* segment. Microsatellite marker analysis of the parental strains, NZO and SJL, revealed a complex pattern of polymorphic and non-polymorphic blocks (Figure 2a).

The peak region of the LOD score curve *(D5Mit82 - D5Mit15)* spans about 8 Mb, including a block of ~3 Mb where the parental strains, NZO and SJL showed microsatellite fragment length polymorphism for five adjacent markers, *D5Mit302, D5Mit256, D5Mit200, D5Mit303,* and *D5Mit290* (Figure 2a). According to NCBI's *Mus musculus* build 36 assembly, a total of seven protein coding genes *(G6pd2, Centd1, 3110047P20Rik, 0610040J01Rik, AA536743, Pgm1, and Tbc1d1)* and one pseudogene *(Cbfa2t2h-ps1)* map to this polymorphic block (Figure 2b). Together with the 12 protein coding genes of the two adjacent proximal and distal non-polymorphic blocks *(Pcdh7, Klf3, Tlr1, Tlr6, 9130005N14Rik, Tmem156, Klhl5, Wdr19, Recc1, Klb, Rpl9, Lias)* a total of 19 genes map into the about 8 Mb *Nob1* peak region (58Mb-65.7 Mb).

In addition to sequencing the coding regions of these 19 candidate genes, we performed expression profiling of 302 genes in the entire *Nob1* QTL (about 100 genes within or close to the *Nob1*.24 segment) with a custom-made macroarray and RNA from various tissues of the two parental strains, including WAT, BAT, skeletal muscle, liver, and hypothalamus (see Methods). Differential gene expression was verified by consecutive quantitative real time PCR. Of all the genes in the *Nob1.24* segment analyzed, only two transcripts were found to be differentially expressed between the two strains in at least one tissue. A gene for a RIKEN cDNA, 2310045A20Rik, which maps close to *D5Mit132,* i.e. 10 Mb proximal to the LOD peak, was up-regulated in brown fat tissue and liver from SJL mice. In contrast, *Tbc1d1* which maps exactly to the polymorphic peak region of the QTL (Figure 2b), was found to be down-regulated 3-fold in skeletal muscle from the lean SJL mice.

Cloning and sequencing of the cDNA for *Tbc1d1* revealed a SJL-specific 7 bp deletion in exon 18 (A⁴⁰⁴⁸ - T⁴⁰⁵⁴ in Genbank Accession no. AK122445; 4048delACTCGCT), which was absent in SJL-related (SWR and FVB) as well as in Non-related mouse strains, including C57BL/6J, NZO, NZB, NON, AKR and SM, respectively (Figure 3a). This deletion results in a frame shift and premature termination of the protein. The inventors confirmed the expression of a truncated protein by *in vitro* translation of wild type and SJL derived cDNA (Figure 3b). *Tbc1d1* (~192 kbp; 22 exons; Figure 3a) belongs to the tre-2/USP6, BUB2, cdcl6 domain family and shows high amino acid sequence homology (~60%) to the insulin signalling protein Tbcld4, also known as AS 160 (Bernards, A. GAPs galore! A survey of putative Ras superfamily GTPase activating proteins in man and Drosophila. Biochim Biophys Acta 1603, 47-82 (2003). Kane, S. et al. A method to identify serine kinase substrates. Akt phosphorylates a novel adipocyte protein with a Rab GTPase-activating protein (GAP) domain. J Biol Chem 277, 22115-8 (2002).). The >5000 bp mRNA for *Tbc1d1* codes for a about 130 kDa protein containing two N-terminal phosphotyrosine-binding (PTB) domains, and a C-terminal GTPase-activating (Rab GAP) domain (Figure 3a). Analysis of the crystal structure of the catalytic domain of Gyp1p, a GAP for Ypt proteins, the yeast homologues of Rab proteins, and a high degree of sequence conservation in yeast and mammalian TBC proteins suggest a common catalytic mechanism by which TBC-domain GAPs may accelerate GTP hydrolysis by a 'dual-finger' mechanism (Pan, X., Eathiraj, S., Munson, M. and Lambright, D.G. TBC-domain GAPs for Rab GTPases accelerate GTP hydrolysis by a dual-finger mechanism. Nature 442, 303-6 (2006)).

Thus, the SJL-specific 7 bp-deletion represents a loss-of-function (LOF) mutation since the mutated protein lacks most invariant residues of the GTPase-activating TBC domain, including the conserved and catalytically essential glutamine finger (YxQ978) (Pan, X., Eathiraj, S., Munson, M. and Lambright, D.G. TBC-domain GAPs for Rab GTPases accelerate GTP hydrolysis by a dual-finger mechanism. Nature 442, 303-6 (2006)).

Sequence analysis of the other 18 genes in the about 8 Mb *Nob1* peak region *(Pcdh7, G6pd2, Centd1, 3110047P20Rik, 0610040JOIRik, AA536743, Pgm1,Klf3, Tlrl, Tlr6, 9130005N14Rik, Tmem156, Klhl5, Wdr19, Recc1, Klb, Rpl9, Lias)* yielded a total of 13 coding SNPs in 8 genes (7 non-synonymous polymorphisms in 5 genes) but none specific for the SJL strain and/or likely to impair protein function (Table 1).

Very recently, it has been shown that the Rab specificity of the Tbc1d1 GAP domain *in vitro* is identical to that of *Tbc1d4* (AS 160), and that upon insulin stimulation Tbc1d1 becomes phosphorylated at a potential AKT consensus site which is conserved between Tbc1d1 and AS 160. In the present study, we used purified recombinant AKT2 from Baculovirus-infected insect cells to confirm that T590 in Tbc1d1, corresponding to T649 in AS160, is a direct target of AKT2 and the only site in Tbc1d1 phosphorylated by this kinase (Figure 6).

In C57BL/6J and NZO mice *Tbc1d1* is expressed mainly in skeletal muscle, heart, pancreas, kidney and hypothalamus, and only low levels of expression were detected in white and brown adipose tissue, liver, intestine and colon (Figure 4a).

However, in the respective tissues from SJL mice the amount of mRNA for *Tbc1d1* was markedly reduced (Figure 4a). Since no sequence variations were detectable in the about 1.5 kb 5'-flanking region of the *Tbc1d1* gene of SJL (Table 1), we assume that mRNA levels of *Tbc1d1* might be reduced by nonsense-mediated mRNA decay (NMD).

In C57BL/6J and NZO mice, we detected two distinct transcripts of *Tbc1d1* that reflect tissue-specific differential splicing of exons 12 and 13. Quantification of the two splice variants which differ by 93 amino acids revealed that the long isoform of *Tbc1d1* is predominantly expressed in muscle and hypothalamus whereas kidney and pancreas express almost exclusively the short isoform (Figure 7).

The inventors next examined whether *Tbc1d1* expression in different tissues is regulated by the fat content of the diet. In skeletal muscle of C57BL6/J and NZO mice, expression of *Tbc1d1* is significantly increased ~3-fold in response to high fat diet, whereas no significant changes were observed in the other tissues (Figure 4b). As a result, in skeletal muscle from animals raised on a high-fat diet, the levels of *Tbc1d1* mRNA are increased by about an order of magnitude in NZO mice compared to SJL mice, respectively. Nevertheless, expression levels of *Tbc1d1* were similar in NZO mice, C57BL6/J and leptin deficient ob/ob mice (B6.V-Lep^{*ob*/*ob*}), indicating that *Tbc1d1* expression *per se* does not correlate with body weight.

A recent study presented genetic evidence for linkage of a *TBC1D1* variant (R125W; allele frequency of 0.09) to obesity predisposition in selected human families20. Human *TBC1D1* is highly homologous to mouse *Tbc1d1,* maps to 4p15-p14 and is also expressed predominantly in skeletal muscle and heart. The biological function of *Tbc1d1* and its possible involvement in insulin signalling however is still unknown.

Overexpression of Tbc1d1 in 3T3-L1 adipocytes (which contain only minute amounts of the endogenous mRNA) has been shown to inhibit insulin-stimulated GLUT4 translocation. However, diabetes-resistant SJL mice which naturally lack functional Tbc1d1 show normal glucose tolerance which argues against a major role of Tbc1d1 for insulin-regulated glucose uptake (as at least mentioned in Stone et al, see above).

Taking all together, *Tbc1d1* is the only gene in the QTL peak region with an SJL specific mutation. This mutation disrupts the protein sequence, strongly suggesting that the 4048delACTCGCT mutation is the causal defect underlying the *Nob1* QTL.

Furthermore, the genetic interaction of *Nob1* with dietary fat, and its function as an obesity suppressor in SJL mice coincides with the differential expression of *Tbc1d1* in response to HFD. The inventors propose that *Tbc1d1* is involved in a yet unknown pathway that regulates lipid metabolism and energy homeostasis in skeletal muscle.

### Methods

### Animals and animal procedures.

The principles of laboratory animal care (NIH Publication No. 85-23) were followed and the study was approved by the Animal Experimentation Ethics Committee of the State Ministry of Agriculture, Nutrition and Forestry (State of Brandenburg, Germany). SJL (SJL/NBom) and NZO mice (NZO/HlBom) were from the inventors' own colony. C57BL/6J (C57BL/6JCr1) mice were obtained from Charles River Laboratories (Sulzfeld, Germany). The recombinant congenic strain B6.SJL-*Nob1*.24 was generated by 3-fold backcross of SJL mice to the C57BL/6J strain via marker selection for *Nob1 (D5Mit290).* N2-backcross of C57BL/6cSJL-*Nob1* to the NZO strain was performed as a reporter cross to generate obese mouse lines on a polygenic background. After weaning (3 weeks of age), mice received low fat (Altromin, Lage, Germany; No. 1314, with 5%, 48%, and 22.5% fat, carbohydrates, and protein, respectively; 12.5 kJ/g) or high fat (No. C1057, with 16.0%, 46.8%, 17.1% fat, carbohydrates, and protein, respectively; 15.4 kJ/g) rodent chow. Throughout the study, mice had free access to food and water. Three to six mice were kept per cage (Macrolon, type III) in a temperature-controlled room (20°C, 55±5% relative humidity) with a 12 h light-dark cycle and lights on at 6 am. Blood glucose was measured weekly under non-fasting conditions in blood samples collected from the tail vein with an Ascensia ELITE® blood glucose meter (Bayer Health Care, Leverkusen, Germany).

### Genotyping and QTL analysis.

DNA was prepared from mice tails with a DNA isolation kit (InViTek, Berlin, Germany). Animals were genotyped for polymorphic microsatellite markers by PCR with oligonucleotide primers obtained from MWG (Ebersberg, Germany), and microsatellite length was determined by non-denaturing polyacrylamide gel electrophoresis (Table 2). Genotyping of SNPs was done by DNA sequencing.

### DNA sequencing and cloning.

The coding regions of candidate genes in the *Nob1* peak region were sequenced after amplification of the corresponding exons (including about 100 bp flanking regions) from genomic DNA with Phusion Polymerase (NEB) or amplification and/or cloning of the cDNAs based on sequence information of NCBI's reference assembly for C57BL/6J build 36 (Table 1). RNA was extracted from flash-frozen tissues using RNeasy-mini columns (Qiagen, Germany) and cDNAs were synthesized from 2 µg of total RNA using the SuperScriptIII first-strand cDNA synthesis kit (Invitrogen) and oligo-dT primers. Sequences were obtained with an ABI 3130×1 sequencer and sequence analysis was performed by aligning the sequence to the genomic C57BL/6J sequence using SeqScape software 2.5 (Applied Biosystems). All exonic changes were verified by resequencing.

The cDNAs for the long (AK122445) and short (AK154591) isoforms of mouse *Tcb1d1* from skeletal muscle and white adipose tissue of C57BL/6J, NZO and SJL mice were amplified using Phusion Polymerase, cloned into pCR2.1-TOPO vector and sequenced. Nucleotide and amino acid positions in *Tbc1d1* refer to the long isoform.

For construction of the Tbc1d1-T590A mutant, the inventors used the Quick-Change method (Stratagene). Resequencing of the (4048delACTCGCT) deletion in exon 18 was performed on the genomic level with the following mouse strains: AKR/J, C57BL/6J, FVB/N, NON/LtJ, NZB/OlaHsd, NZO/HlBom, SJL/NBom, SM/JCrl, and SWR/J.

### Expression profiling and quantitative real time PCR.

Gene expression profiling using custom made cDNA macroarrays was performed as described (Herwig, R., Aanstad, P., Clark, M. and Lehrach, H. Statistical evaluation of differential expression on cDNA nylon arrays with replicated experiments. *Nucleic Acids Res* **29**, E117 (2001)). Briefly, 10 ng purified PCR probes (about 500 bp) derived from 306 cDNAs of the *Nob1* region were immobilized on nylon filter membranes and hybridized with [α- ³³P]DCTP labelled cDNA from various tissues of NZO and SJL mice, including BAT, WAT, liver, skeletal muscle, intestine, and hypothalamus. For hybridization, cDNA was synthesized from 10 µg of total RNA using the SuperScriptIII first-strand cDNA synthesis kit and oligo-dT primers. After hybridization the filter membranes were exposed to phosphorimager screens for 14-16 h, and data from 2-3 independent replicates of each hybridization were used to assess expression differences between the two strains. Strain-dependent differential expression was verified by quantitative SYBR Green real time PCR using a 7300 Real-Time PCR System (Applied Biosystems). Measurements of Tbc1d1 expression was performed with TaqMan RT-PCR; Primers (Supplementary Table 2) were obtained from MWG.

### Protein expression and phosphorylation of Tbc1d1.

Full-*lengthTbc1*d*1* (short isoform) from C57BL/6J and SJL mice was *in vitro* translated using TNT T7 Quick Coupled Transcription/Translation System (Promega) and [35S]methionine (10 Ci/µl; Hartmann Analytics) according to the manufacturer's instruction. For kinase assays, the long form of *Tbc1d1* containing a N-terminal triple FLAG tag was overexpressed in HeLa cells by Lipofectamine (Invitrogen) transfection and immunoprecipitated using a monoclonal FLAG antibody (Sigma). In vitro phosphorylation of FLAGTbcld1 and the T590A mutant was using recombinant AKT2 kinase was carried out as described 22. Briefly, the substrates were incubated with affinity-purified, activated AKT2 kinase from *Sf9* cells in kinase buffer (50 mM Tris-HCl pH 7.5, 15 mM Mg-acetate, 5 mM glycerophosphate, 0.2 mM sodium vanadate, 1 mM EGTA), 100 µM [γ32P]ATP for 15 min at RT, and the phosphate incorporation into the proteins was determined after SDS-PAGE and Phosphorimager analysis.

**Accession numbers.** GenBank, EntrezID:. *0610040J01Rik,* 76261; *3110047P20Rik, 319807; 9130005N14Rik,* 68303; *Aa536743,* 100532; *Centd1,* 212285; *G6pd2,* 14380; *Klb,* 83379; *Klf3,* 16599; *Klh15,* 71778; *Lias,* 79464; *Pcdh7,* 54216; *Pgm1,* 66681; *Recc1,,* 19687; *Rp19,* 20005; *Tbc1d1d,* 57915; *Tlr1,* 21897; *Tlr6,* 21899; *Tmem156,* 243025; *Ugdh,* 22235; *Wdr19,* 213081.

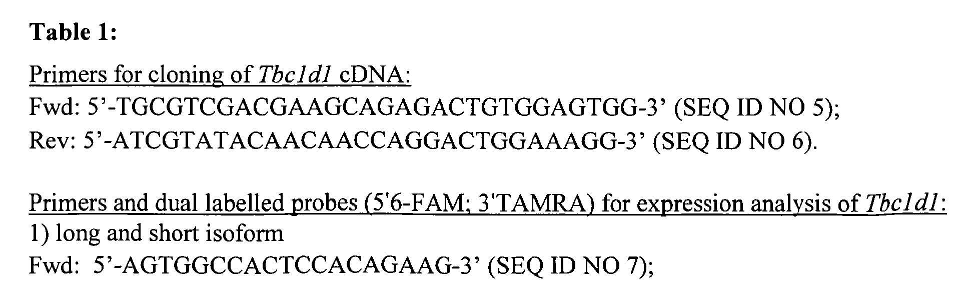

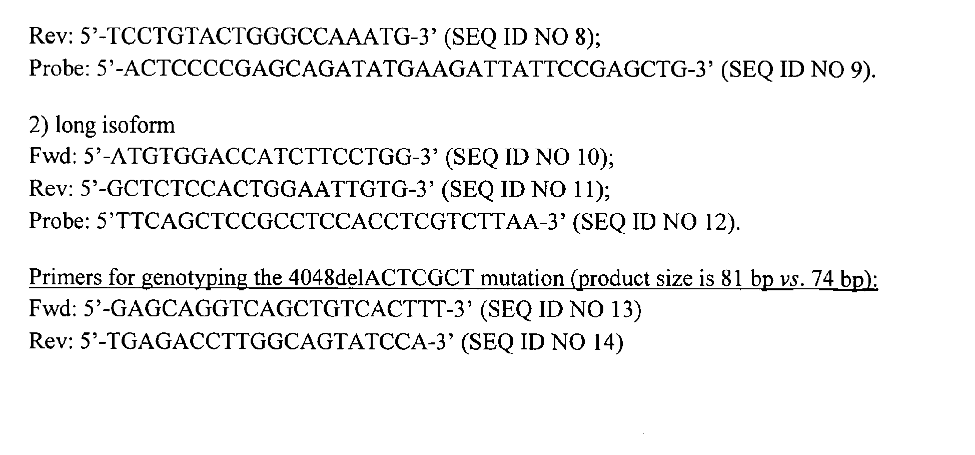

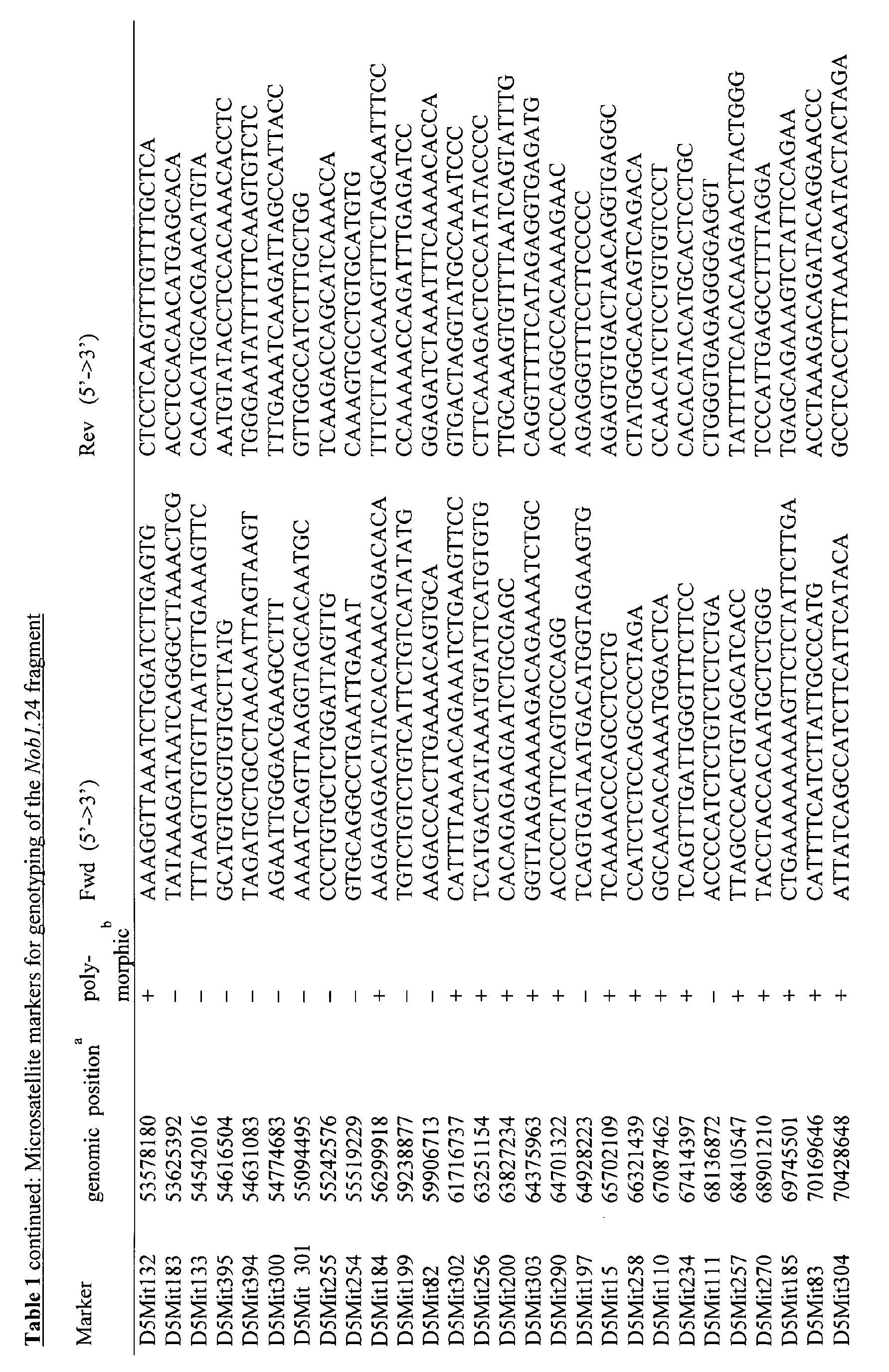

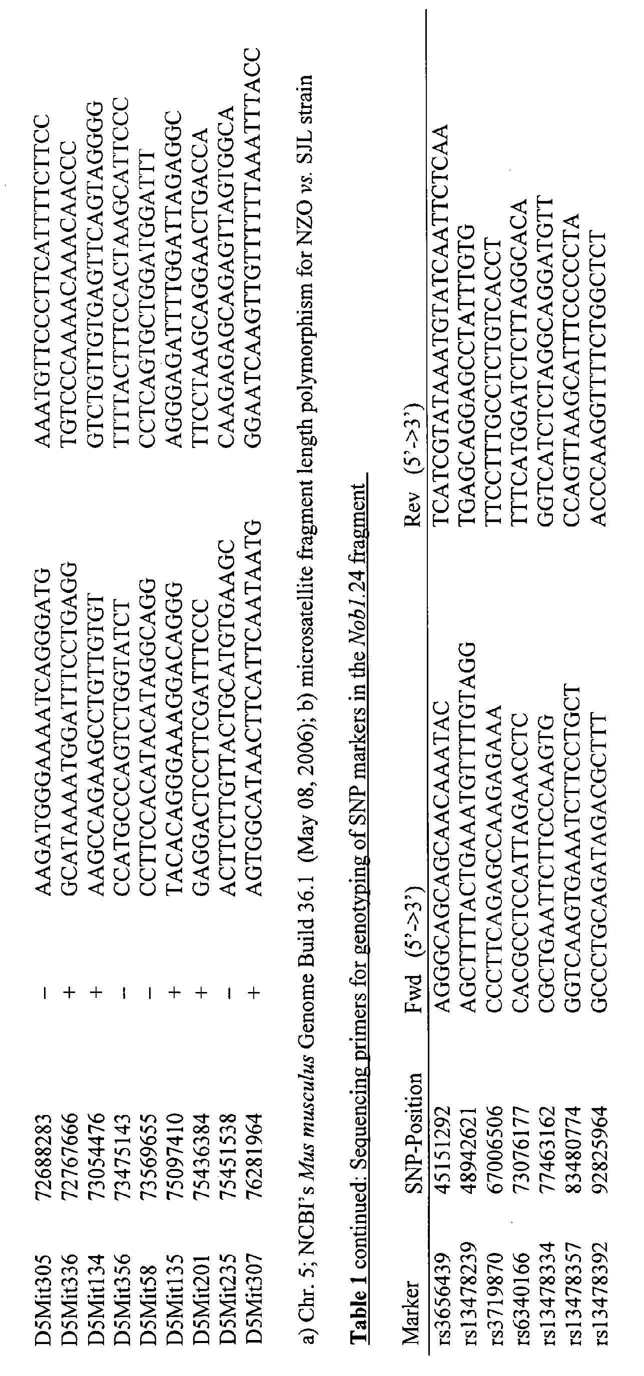

## Claims

1. A method for diagnosing obesity or diabetes or an increased risk for obesity or diabetes in a mammal, comprising analysis of the biological activity and/or expression of TBC1D1 in a biological sample obtained from said mammal to be diagnosed, wherein a lack or reduced expression of the gene for TBC1D1 or a lack or reduced biological activity of TBC1D1 in said mammal, when compared to a biological sample obtained from a non-diseased mammal, is indicative for an absence or a reduced risk for obesity or diabetes in said mammal.

2. The method according to claim 1, wherein said obesity or diabetes is dietary fat-related.

3. The method according to claim 1 or 2, wherein said mammal is a mouse, rat or human.

4. The method according to any of claims 1 to 3, wherein said biological sample contains biological material that is derived from skeletal muscle, heart, pancreas, kidney, and/or hypothalamus of said mammal to be diagnosed.

5. The method according to any of claims 1 to 5, wherein said diagnoses comprises the use of TBC1D1-specific antibodies, hybridisation analysis, quantitative PCR, mRNA analysis, quantitative analysis of the amount of TBC1D1-protein, sequencing of the TBC1D1-locus, and/or an analysis of the activity of TBC1D1 in said sample.

6. The method according to any of claims 1 to 5, wherein TBC1D1 is down-regulated at least 3-fold in skeletal muscle.

7. The method according to any of claims 1 to 6, wherein the gene for TBC1D1 contains a deletion, in particular a 7 bp deletion in exon 18 (A⁴⁰⁴⁸ - T⁴⁰⁵⁴ Genbank Acc No. AK122445) of the mouse gene for TBC1D1 or the homologous deletion in the human gene for TBC1D1.

8. The method according to any of claims 1 to 7, wherein the biological activity of TBC1D1 is the activity of the Rab-specific GTP-ase activating TBC domain.

9. A screening tool for an agent for treating or preventing obesity or diabetes, wherein said tool is selected from
a) a polynucleotide according to SEQ ID NO: 3 or 4,
b) a polypeptide that is encoded by SEQ ID NO: 3 or 4, in particular having an amino acid sequence of SEQ ID NO: 1 or 2,
c) a polypeptide having an amino acid sequence having a 90% or more homology with an amino acid sequence of SEQ ID NO: 1 or 2, and exhibiting a TBC1D1 activity, and
d) a polypeptide having an amino acid sequence in which 1 to 10 amino acids are deleted, substituted, and/or added in the amino acid sequence according to SEQ ID NO: 1 or 2, and exhibiting a TBC1D1 activity.

10. The screening tool for an agent for treating or preventing obesity or diabetes according to claim 9, wherein said tool is a polypeptide consisting of an amino acid sequence of SEQ ID NO: 1 or 2.

11. A screening tool for an agent for treating or preventing obesity or diabetes, wherein the tool is a cell which is transformed with an expression vector comprising a polynucleotide encoding a polypeptide according to claim 9, and expresses said polypeptide.

12. A screening tool for an agent for treating or preventing obesity or diabetes, wherein the tool is a non-human transgenic mammal overexpressing TBC1D1 and/or carrying a TBC1D1 genetic reporter-construct.

13. A method of identifying a compound that modulate the expression and/or biological activity of TBC1D1 in a cell, comprising the steps of a) contacting a cell expressing TBC1D1 with at least one potentially interacting compound, and b) measuring the modulation of the expression and/or biological activity of TBC1D1 in said cell.

14. The method according to claim 13, wherein said cell is selected from cells of the skeletal muscle, heart, pancreas, kidney, hypothalamus, the cell according to claim 11. and the non-human transgenic mammal according to claim 12.

15. The method according to claim 13 or 14, wherein said compound as identified is an inhibitor of the expression and/or biological activity of TBC1D1 in said cell.

16. The method according to claim 15, wherein said compound is selected from a peptide library, a combinatory library, a cell extract, in particular a plant cell extract, a "small molecular drug", an antibody or fragment thereof, an antisense oligonucleotide, an siRNA, a protein and/or a protein fragment, in particular an antibody recognizing the amino acid sequence TGQPSAPGPRPMRKS.

17. A method for manufacturing a pharmaceutical composition for treating or preventing obesity or diabetes, comprising the steps of: performing a method according to any of claims 13 to 16, and formulating said modulator into a pharmaceutical composition.

18. A pharmaceutical composition for treating or preventing obesity or diabetes, obtainable by a method according to claim 17.

19. A method for treating or preventing obesity or diabetes, comprising a method according to any of claim 1 to 8, and providing a treatment to said mammal based on, at least in part, the result of said diagnosis.

20. A method for treating or preventing obesity or diabetes, comprising administering to a subject in need thereof an effective amount of a pharmaceutical composition according to claim 18.

21. Use of a modulator of the expression and/or the biological activity of TBC1D1 in a cell for the manufacture of a pharmaceutical composition for treating or preventing obesity or diabetes.

22. Use according to claim 21, wherein said obesity or diabetes is dietary fat-related.

23. Use according to claim 21 or 22, wherein said modulator is an inhibitor of the expression and/or biological activity of TBC1D1.


SEQUENCE LISTING

<110> Deutsches Institut für Ernährungsforschung

<120> TBC1D1 as a marker in the response to dietary fat, and obesity

<130> FB18538

<160> 102

<170> PatentIn version 3.4

<210> 1
<211> 1168
<212> PRT
<213> Mus musculus

<400> 1

Met Glu Pro Ile Thr Phe Thr Ala Arg Lys His Leu Leu Ser Asn Glu
1               5                   10                  15

Val Ser Val Asp Phe Gly Leu Gln Leu Val Gly Ser Leu Pro Val His
            20                  25                  30

Ser Leu Thr Thr Met Pro Met Leu Pro Trp Val Val Ala Glu Val Arg
        35                  40                  45

Arg Leu Ser Arg Gln Ser Thr Arg Lys Glu Pro Val Thr Lys Gln Val
    50                  55                  60

Arg Leu Cys Val Ser Pro Ser Gly Leu Arg Cys Glu Pro Glu Pro Gly
65                  70                  75                  80

Arg Ser Gln Gln Trp Asp Pro Leu Ile Tyr Ser Ser Ile Phe Glu Cys
                85                  90                  95

Lys Pro Gln Arg Val His Lys Leu Ile His Asn Ser His Asp Pro Ser
            100                 105                 110

Tyr Phe Ala Cys Leu Ile Lys Glu Asp Ala Val His Arg Gln Ser Ile
        115                 120                 125

Cys Tyr Val Phe Lys Ala Asp Asp Gln Thr Lys Val Pro Glu Ile Ile
    130                 135                 140

Ser Ser Ile Arg Gln Ala Gly Lys Ile Ala Arg Gln Glu Glu Leu His
145                 150                 155                 160

Cys Pro Ser Glu Phe Asp Asp Thr Phe Ser Lys Lys Phe Glu Val Leu
                165                 170                 175

Phe Cys Gly Arg Val Thr Val Ala His Lys Lys Ala Pro Pro Ala Leu
            180                 185                 190

Ile Asp Glu Cys Ile Glu Lys Phe Asn His Val Ser Gly Ser Arg Gly
        195                 200                 205

Ser Glu Ser Pro Arg Pro Asn Pro Pro His Ala Ala Pro Thr Gly Ser
    210                 215                 220

Gln Glu Pro Val Arg Arg Pro Met Arg Lys Ser Phe Ser Gln Pro Gly
225                 230                 235                 240

Leu Arg Ser Leu Ala Phe Arg Lys Glu Leu Gln Asp Gly Gly Leu Arg
                245                 250                 255

Ser Ser Gly Phe Phe Ser Ser Phe Glu Glu Ser Asp Ile Glu Asn His
            260                 265                 270

Leu Ile Ser Gly His Asn Ile Val Gln Pro Thr Asp Ile Glu Glu Asn
        275                 280                 285

Arg Thr Met Leu Phe Thr Ile Gly Gln Ser Glu Val Tyr Leu Ile Ser
    290                 295                 300

Pro Asp Thr Lys Lys Ile Ala Leu Glu Lys Asn Phe Lys Glu Ile Ser
305                 310                 315                 320

Phe Cys Ser Gln Gly Ile Arg His Val Asp His Phe Gly Phe Ile Cys
                325                 330                 335

Arg Glu Ser Ser Gly Gly Gly Gly Phe His Phe Val Cys Tyr Val Phe
            340                 345                 350

Gln Cys Thr Asn Glu Ala Leu Val Asp Glu Ile Met Met Thr Leu Lys
        355                 360                 365

Gln Ala Phe Thr Val Ala Ala Val Gln Gln Thr Ala Lys Ala Pro Ala
    370                 375                 380

Gln Leu Cys Glu Gly Cys Pro Leu Gln Ser Leu His Lys Leu Cys Glu
385                 390                 395                 400

Arg Ile Glu Gly Met Asn Ser Ser Lys Thr Lys Leu Glu Leu Gln Lys
                405                 410                 415

His Leu Thr Thr Leu Thr Asn Gln Glu Gln Ala Thr Ile Phe Glu Glu
            420                 425                 430

Val Gln Lys Leu Arg Pro Arg Asn Glu Gln Arg Glu Asn Glu Leu Ile
        435                 440                 445

Ile Ser Phe Leu Arg Cys Leu Tyr Glu Glu Lys Gln Lys Glu His Ile
    450                 455                 460

His Ile Gly Glu Met Lys Gln Thr Ser Gln Met Ala Ala Glu Asn Ile
465                 470                 475                 480

Gly Ser Glu Leu Pro Pro Ser Ala Thr Arg Phe Arg Leu Asp Met Leu
                485                 490                 495

Lys Asn Lys Ala Lys Arg Ser Leu Thr Glu Ser Leu Glu Ser Ile Leu
            500                 505                 510

Ser Arg Gly Asn Lys Ala Arg Gly Leu Gln Glu His Ser Ile Ser Val
        515                 520                 525

Asp Leu Asp Ser Ser Leu Ser Ser Thr Leu Ser Asn Thr Ser Lys Glu
    530                 535                 540

Pro Ser Val Cys Glu Lys Glu Ala Leu Pro Ile Ser Glu Ser Ser Phe
545                 550                 555                 560

Lys Leu Leu Gly Ser Ser Glu Asp Leu Ser Ser Asp Ser Glu Ser His
                565                 570                 575

Leu Pro Glu Glu Pro Ala Pro Leu Ser Pro Gln Gln Ala Phe Arg Arg
            580                 585                 590

Arg Ala Asn Thr Leu Ser His Phe Pro Ile Glu Cys Gln Glu Pro Pro
        595                 600                 605

Gln Pro Ala Arg Gly Ser Pro Gly Val Ser Gln Arg Lys Leu Met Arg
    610                 615                 620

Tyr His Ser Val Ser Thr Glu Thr Pro His Glu Arg Lys Asp Phe Glu
625                 630                 635                 640

Ser Lys Ala Asn His Leu Gly Asp Ser Gly Gly Thr Pro Val Lys Thr
                645                 650                 655

Arg Arg His Ser Trp Arg Gln Gln Ile Phe Leu Arg Val Ala Thr Pro
            660                 665                 670

Gln Lys Ala Cys Asp Ser Ser Ser Arg Tyr Glu Asp Tyr Ser Glu Leu
        675                 680                 685

Gly Glu Leu Pro Pro Arg Ser Pro Leu Glu Pro Val Cys Glu Asp Gly
    690                 695                 700

Pro Phe Gly Pro Pro Pro Glu Glu Lys Lys Arg Thr Ser Arg Glu Leu
705                 710                 715                 720

Arg Glu Leu Trp Gln Lys Ala Ile Leu Gln Gln Ile Leu Leu Leu Arg
                725                 730                 735

Met Glu Lys Glu Asn Gln Lys Leu Gln Ala Ser Glu Asn Asp Leu Leu
            740                 745                 750

Asn Lys Arg Leu Lys Leu Asp Tyr Glu Glu Ile Thr Pro Cys Leu Lys
        755                 760                 765

Glu Val Thr Thr Val Trp Glu Lys Met Leu Ser Thr Pro Gly Arg Ser
    770                 775                 780

Lys Ile Lys Phe Asp Met Glu Lys Met His Ser Ala Val Gly Gln Gly
785                 790                 795                 800

Val Pro Arg His His Arg Gly Glu Ile Trp Lys Phe Leu Ala Glu Gln
                805                 810                 815

Phe His Leu Lys His Gln Phe Pro Ser Lys Gln Gln Pro Lys Asp Val
            820                 825                 830

Pro Tyr Lys Glu Leu Leu Lys Gln Leu Thr Ser Gln Gln His Ala Ile
        835                 840                 845

Leu Ile Asp Leu Gly Arg Thr Phe Pro Thr His Pro Tyr Phe Ser Ala
    850                 855                 860

Gln Leu Gly Ala Gly Gln Leu Ser Leu Tyr Asn Ile Leu Lys Ala Tyr
865                 870                 875                 880

Ser Leu Leu Asp Gln Glu Val Gly Tyr Cys Gln Gly Leu Ser Phe Val
                885                 890                 895

Ala Gly Ile Leu Leu Leu His Met Ser Glu Glu Glu Ala Phe Lys Met
            900                 905                 910

Leu Lys Phe Leu Met Phe Asp Met Gly Leu Arg Lys Gln Tyr Arg Pro
        915                 920                 925

Asp Met Ile Ile Leu Gln Ile Gln Met Tyr Gln Leu Ser Arg Leu Leu

930                 935                 940

His Asp Tyr His Arg Asp Leu Tyr Asn His Leu Glu Glu His Glu Ile
945                 950                 955                 960

Gly Pro Ser Leu Tyr Ala Ala Pro Trp Phe Leu Thr Met Phe Ala Ser
                965                 970                 975

Gln Phe Pro Leu Gly Phe Val Ala Arg Val Phe Asp Met Ile Phe Leu
            980                 985                 990

Gln Gly Thr Glu Val Ile Phe Lys  Val Ala Leu Ser Leu  Leu Gly Ser
        995                 1000                1005

His Lys  Pro Leu Ile Leu Gln  His Glu Asn Leu Glu  Thr Ile Val
    1010                 1015                 1020

Asp Phe  Ile Lys Ser Thr Leu  Pro Asn Leu Gly Leu  Val Gln Met
    1025                 1030                 1035

Glu Lys  Thr Ile Asn Gln Val  Phe Glu Met Asp Ile  Ala Lys Gln
    1040                 1045                 1050

Leu Gln  Ala Tyr Glu Val Glu  Tyr His Val Leu Gln  Glu Glu Leu
    1055                 1060                 1065

Ile Asp  Ser Ser Pro Leu Ser  Asp Asn Gln Arg Met  Asp Lys Leu
    1070                 1075                 1080

Glu Lys  Thr Asn Ser Ser Leu  Arg Lys Gln Asn Leu  Asp Leu Leu
    1085                 1090                 1095

Glu Gln  Leu Gln Val Ala Asn  Gly Arg Ile Gln Ser  Leu Glu Ala
    1100                 1105                 1110

Thr Ile  Glu Lys Leu Leu Ser  Ser Glu Ser Lys Leu  Lys Gln Ala
    1115                 1120                 1125

Met Leu  Thr Leu Glu Leu Glu  Arg Ser Ala Leu Leu  Gln Thr Val
    1130                 1135                 1140

Glu Glu  Leu Arg Arg Arg Ser  Ala Glu Pro Ser Asp  Arg Glu Pro
    1145                 1150                 1155

Glu Cys  Thr Gln Pro Glu Pro  Thr Gly Asp
    1160                 1165

<210>  2
<211>  1266
<212>  PRT
<213>  Homo sapiens

<400>  2

His Val Phe Leu Ala Leu Gln Thr Ser Pro Arg Met Glu Ala Ile Thr
1               5                   10                  15

Phe Thr Ala Arg Lys His Pro Phe Pro Asn Glu Val Ser Val Asp Phe
            20                  25                  30

Gly Leu Gln Leu Val Gly Ser Leu Pro Val His Ser Leu Thr Thr Met
        35                  40                  45

Pro Met Leu Pro Trp Val Val Ala Glu Val Arg Arg Leu Ser Gly Gln
    50                  55                  60

Cys Ser Lys Lys Glu Pro Arg Thr Lys Gln Val Arg Leu Trp Val Ser
65                  70                  75                  80

Pro Ser Gly Leu Arg Cys Glu Pro Asp Leu Glu Lys Ser Gln Pro Trp
                85                  90                  95

Asp Pro Leu Ile Cys Ser Ser Ile Phe Glu Cys Lys Pro Gln Arg Val
            100                 105                 110

His Lys Leu Ile His Asn Ser His Asp Pro Ser Tyr Phe Ala Cys Leu
        115                 120                 125

Ile Lys Glu Asp Ala Ala His Arg Gln Ser Leu Cys Tyr Val Phe Lys
    130                 135                 140

Ala Asp Asp Gln Thr Lys Val Pro Glu Ile Ile Ser Ser Ile Arg Gln
145                 150                 155                 160

Ala Gly Lys Ile Ala Arg Gln Glu Glu Leu Arg Cys Pro Ser Glu Phe
                165                 170                 175

Asp Asp Thr Phe Ala Lys Lys Phe Lys Val Leu Phe Cys Gly Arg Val
            180                 185                 190

Thr Val Ala His Lys Lys Ala Pro Pro Ala Leu Ile Asp Glu Cys Ile
        195                 200                 205

Glu Lys Phe Asn His Val Ser Cys Gly Arg Arg Thr Asp Trp Glu Ala
    210                 215                 220

Pro Thr Gly Gln Pro Ser Ala Pro Gly Pro Arg Pro Met Arg Lys Ser
225                 230                 235                 240

Phe Ser Gln Pro Gly Leu Arg Ser Leu Ala Phe Arg Lys Glu Phe Gln
                245                 250                 255

Asp Ala Ser Leu Arg Ser Ser Thr Phe Ser Ser Phe Asp Asn Asp Ile
            260                 265                 270

Glu Asn His Leu Ile Gly Gly His Asn Val Val Gln Pro Thr Asp Met
        275                 280                 285

Glu Glu Asn Arg Thr Met Leu Phe Thr Ile Gly Gln Ser Glu Val Tyr
    290                 295                 300

Leu Ile Ser Pro Asp Thr Lys Lys Ile Ala Leu Glu Lys Asn Phe Lys
305                 310                 315                 320

Glu Ile Ser Phe Cys Ser Gln Gly Ile Arg His Val Asp His Phe Gly
                325                 330                 335

Phe Ile Cys Arg Glu Cys Ser Gly Gly Gly Ser Gly Gly Phe His Phe
            340                 345                 350

Val Cys Tyr Val Phe Gln Cys Thr Asn Glu Ala Leu Val Asp Glu Ile
        355                 360                 365

Met Met Thr Leu Lys Gln Ala Phe Thr Val Ala Ala Val Gln Gln Thr
    370                 375                 380

Ala Lys Ala Pro Ala Gln Leu Cys Glu Gly Cys Pro Leu Gln Gly Leu
385                 390                 395                 400

His Lys Leu Cys Glu Arg Ile Glu Gly Met Asn Ser Ser Lys Thr Lys
                405                 410                 415

Leu Glu Leu Gln Lys His Leu Thr Thr Leu Thr Asn Gln Glu Gln Ala
            420                 425                 430

Thr Ile Phe Glu Glu Val Gln Lys Leu Arg Pro Arg Asn Glu Gln Arg
        435                 440                 445

Glu Asn Glu Leu Ile Ile Ser Phe Leu Arg Cys Leu Tyr Glu Glu Lys
    450                 455                 460

Gln Lys Glu His Ser His Thr Gly Glu Pro Lys Gln Thr Leu Gln Val
465                 470                 475                 480

Ala Ala Glu Asn Ile Gly Ser Asp Leu Pro Pro Ser Ala Ser Arg Phe
                485                 490                 495

Arg Leu Asp Ser Leu Lys Asn Arg Ala Lys Arg Ser Leu Thr Glu Ser
            500                 505                 510

Leu Glu Ser Ile Leu Ser Arg Gly Asn Lys Ala Arg Gly Leu Gln Asp
        515                 520                 525

His Ser Ala Ser Val Asp Leu Asp Ser Ser Thr Ser Ser Thr Leu Ser
    530                 535                 540

Asn Thr Ser Lys Glu Leu Ser Met Gly Asp Lys Glu Ala Phe Pro Val
545                 550                 555                 560

Ser Glu Thr Ser Phe Lys Leu Leu Gly Ser Ser Asp Asp Leu Ser Ser
                565                 570                 575

Asp Ser Glu Gly His Ile Ala Glu Glu Ser Ala Leu Leu Ser Pro Gln
            580                 585                 590

Gln Ala Phe Arg Arg Arg Ala Asn Thr Leu Ser His Phe Pro Val Glu
        595                 600                 605

Cys Pro Ala Pro Pro Glu Pro Ala Gln Ser Ser Pro Gly Val Ser Gln
    610                 615                 620

Arg Lys Leu Met Arg Tyr His Ser Val Ser Thr Glu Thr Pro His Glu
625                 630                 635                 640

Arg Asn Val Asp His Leu Pro Gly Gly Glu Ser Gln Gly Cys Pro Gly
                645                 650                 655

Gln Pro Ser Ala Pro Pro Pro Pro Arg Leu Asn Pro Ser Ala Ser Ser
            660                 665                 670

Pro Asn Phe Phe Lys Tyr Leu Lys His Asn Ser Ser Gly Glu Gln Ser
        675                 680                 685

Gly Asn Ala Val Pro Lys Ser Val Ser Tyr Arg Asn Ala Leu Arg Lys
    690                 695                 700

Lys Leu His Ser Ser Ser Ser Val Pro Asn Phe Leu Lys Phe Leu Ala
705                 710                 715                 720

Pro Val Asp Glu Asn Asn Thr Cys Asp Phe Lys Asn Thr Asn Arg Asp
                725                 730                 735

Phe Glu Ser Lys Ala Asn His Leu Gly Asp Thr Asp Gly Thr Pro Val
            740                 745                 750

Lys Thr Arg Arg His Ser Trp Arg Gln Gln Ile Phe Leu Arg Val Ala
        755                 760                 765

Thr Pro Gln Lys Ala Cys Asp Ser Pro Ser Arg Tyr Glu Asp Tyr Ser
    770                 775                 780

Glu Leu Gly Glu Leu Pro Pro Arg Ser Pro Leu Glu Pro Val Cys Glu
785                 790                 795                 800

Asp Gly Pro Phe Gly Pro Val Gln Glu Glu Lys Arg Lys Thr Ser Arg
                805                 810                 815

Glu Leu Arg Glu Leu Trp Lys Lys Ala Ile Leu Gln Gln Ile Leu Leu
            820                 825                 830

Leu Arg Met Glu Lys Glu Asn Gln Lys Leu Gln Ala Ser Glu Asn Asp
        835                 840                 845

Leu Leu Asn Lys Arg Leu Lys Leu Asp Tyr Glu Glu Ile Thr Pro Cys
    850                 855                 860

Leu Lys Glu Val Thr Thr Val Trp Glu Lys Met Leu Ser Thr Pro Gly
865                 870                 875                 880

Arg Ser Lys Ile Lys Phe Asp Met Glu Lys Val His Ser Ala Val Gly
                885                 890                 895

Gln Gly Val Pro Arg His His Arg Gly Glu Ile Trp Lys Phe Leu Ala
            900                 905                 910

Glu Gln Phe His Leu Lys His Pro Phe Pro Ser Lys Gln Gln Pro Lys
        915                 920                 925

Asp Val Pro Tyr Lys Glu Leu Leu Lys Lys Leu Thr Ser Gln Gln His
    930                 935                 940

Ala Ile Leu Ile Asp Leu Gly Arg Thr Phe Pro Thr His Pro Tyr Phe
945                 950                 955                 960

Ser Ala Gln Leu Gly Ala Gly Gln Leu Ser Leu Tyr Asn Ile Leu Lys
                965                 970                 975

Ala Tyr Ser Leu Leu Asp Gln Glu Val Gly Tyr Cys Gln Gly Leu Ser

            980                 985                 990

Phe Val Ala Gly Ile Leu Leu Leu His Met Ser Glu Glu Glu Ala Phe
        995                 1000                1005

Lys Met Leu Lys Phe Leu Met Phe Asp Met Gly Leu Arg Lys Gln
    1010                1015                1020

Tyr Arg Pro Asp Met Ile Ile Leu Gln Ile Gln Met Tyr Gln Leu
    1025                1030                1035

Ser Arg Leu Leu His Asp Tyr His Arg Asp Leu Tyr Asn His Leu
    1040                1045                1050

Glu Glu His Glu Ile Gly Pro Ser Leu Tyr Ala Ala Pro Trp Phe
    1055                1060                1065

Leu Thr Val Phe Ala Ser Gln Phe Pro Leu Gly Phe Val Ala Arg
    1070                1075                1080

Val Phe Asp Met Ile Phe Leu Gln Gly Ser Glu Val Ile Phe Lys
    1085                1090                1095

Val Ala Leu Ser Leu Leu Gly Ser His Lys Pro Leu Ile Leu Gln
    1100                1105                1110

His Glu Asn Leu Glu Thr Ile Val Asp Phe Ile Lys Asn Thr Leu
    1115                1120                1125

Pro Asn Leu Gly Leu Val Gln Met Glu Lys Thr Ile Ser Gln Val
    1130                1135                1140

Phe Glu Met Asp Ile Ala Lys Gln Leu Gln Ala Tyr Glu Val Glu
    1145                1150                1155

Tyr His Val Leu Gln Glu Glu Leu Ile Glu Ser Ser Pro Leu Ser
    1160                1165                1170

Asp Asn Gln Arg Met Glu Lys Leu Glu Lys Thr Asn Ser Ser Leu
    1175                1180                1185

Arg Lys Gln Asn Leu Asp Leu Leu Glu Gln Leu Gln Val Ala Asn
    1190                1195                1200

Ala Arg Ile Gln Ser Leu Glu Ala Thr Val Glu Lys Leu Leu Thr
    1205                1210                1215

Ser Glu Ser Lys Leu Lys Gln Ala Ala Leu Thr Leu Glu Val Glu
    1220                1225                1230

Arg Ser Ala Leu Leu Gln Met Val Glu Glu Leu Arg Arg Gln Ser
    1235                1240                1245

Ala Arg Pro Ser Thr Pro Glu Pro Asp Cys Thr Gln Leu Glu Pro
    1250                1255                1260

Thr Gly Asp
    1265

<210> 3
<211> 5688
<212> DNA
<213> Mus musculus

<400> 3
tgctcctggt gccgccaccg tccgccggtg cctgttgctg ccgccgccgc gggacctgct      60

gtgtcctcag ctgggtggag aagaggcggg cgccgagccg aggggagccc cctccccgtc     120

cccccgcggc gggaagagcg cagccagccg ggtgcgatgg actccccgcc cgcccaggcc     180

gtccccagga tgcccccaag cacctgcgcg tcccggcccg gccccgggct ctgagcgcgc     240

cgcggcacag gtttctgcat atgaagtgtg taaaatagat tgcttgatcc aaaacagaaa     300

aacagtgata actgttttgc tgagttccca gacccttccc aagatggaac caataacatt     360

cacagcaagg aaacatctgc tttctaacga ggtctcggtg gattttggcc tgcagctggt     420

gggctccctg cctgtgcatt ccctgaccac catgcccatg ctgccctggg ttgtggctga     480

ggtgcgaaga ctcagcaggc agtccaccag aaaggaacct gtaaccaagc aagtccggct     540

ttgcgtttca ccctctggac tgagatgtga acctgagcca gggagaagtc aacagtggga     600

tcccctgatc tattccagca tctttgagtg caagcctcag cgtgttcaca aactgattca     660

caacagtcat gacccaagtt actttgcttg tctgattaag gaagacgctg tccaccggca     720

gagtatctgc tatgtgttca aagccgatga tcaaacaaaa gtgcctgaga tcatcagctc     780

catccgtcag gcggggaaga tcgcccggca ggaggagctg cactgcccgt ccgagttcga     840

cgacacgttt tccaagaagt tcgaggtgct cttctgcggc cgcgtgacgg tggcgcacaa     900

gaaggctccg ccggccctga tcgacgagtg catcgagaag ttcaatcacg tcagcggcag     960

ccgggggtcc gagagccccc gccccaaccc gccccatgcc gcgcccacag ggagccagga    1020

gcctgtgcgc aggcccatgc gcaagtcctt ctcccagccc ggcctgcgct cgctggcctt    1080

taggaaggag ctgcaggatg ggggcctccg aagcagcggc ttcttcagct ccttcgagga    1140

gagcgacatt gagaaccacc tcattagcgg acacaatatt gtgcagccca cagatatcga    1200

ggaaaatcga actatgctct tcacgattgg ccagtctgaa gtttacctca tcagtcctga    1260

caccaaaaaa atagcattgg agaaaaattt taaggagata tccttttgct ctcagggcat 1320

cagacacgtg gaccactttg ggtttatctg tcgggagtct tccggaggtg gcggctttca 1380

ttttgtctgt tacgtgtttc agtgcacaaa tgaggctctg gttgatgaaa ttatgatgac 1440

cctgaaacag gccttcacgg tggccgcagt gcagcagaca gctaaggcgc cagcccagct 1500

gtgtgagggc tgccccctgc aaagcctgca caagctctgt gagaggatag agggaatgaa 1560

ttcttccaaa acaaaactag aactgcaaaa gcacctgacg acattaacca atcaggagca 1620

ggcgactatt tttgaagagg ttcagaaatt gagaccgaga aatgagcagc gagagaatga 1680

attgattatt tcttttctga gatgtttata tgaagagaaa cagaaagaac acatccatat 1740

tggggagatg aagcagacat cgcagatggc agcagagaat attggaagtg aattaccacc 1800

cagtgccact cgatttaggc tagatatgct gaaaaacaaa gcaaagagat ctttaacaga 1860

gtctttagaa agtattttgt cccggggtaa taaagccaga ggcctgcagg aacactccat 1920

cagtgtggat ctggatagct ccctgtctag tacattaagt aacaccagca aagagccatc 1980

tgtgtgtgaa aaggaggcct tgcccatctc tgagagctcc tttaagctcc tcggctcctc 2040

ggaggacctg tccagtgact cggagagtca tctcccagaa gagccagctc cgctgtcgcc 2100

ccagcaggcc ttcaggaggc gagcaaacac cctgagtcac ttccccatcg aatgccagga 2160

acctccacaa cctgcccggg ggtccccggg ggtttcgcaa aggaaactta tgaggtatca 2220

ctcagtgagc acagagacgc ctcatgaacg aaaggacttt gaatccaaag caaaccatct 2280

tggtgattct ggtgggactc ctgtgaagac ccggaggcat tcctggaggc agcagatatt 2340

cctccgagta gccaccccgc agaaggcgtg cgattcttcc agcagatatg aagattattc 2400

agagctggga gagcttcccc cacgatctcc tttagaacca gtttgtgaag atgggccctt 2460

tggcccccca ccagaggaaa agaaaaggac atctcgtgag ctccgagagc tgtggcaaaa 2520

ggctattctt caacagatac tgctgcttag aatggagaag gaaaatcaga agctccaagc 2580

ctctgaaaat gatttgctga acaagcgcct gaagctcgat tatgaagaaa ttactccctg 2640

tcttaaagaa gtaactacag tgtgggaaaa gatgcttagc actccaggaa gatcaaaaat 2700

taagtttgac atggaaaaaa tgcactcggc tgttgggcaa ggtgtgccac gtcatcaccg 2760

aggtgaaatc tggaaatttc tagctgagca attccacctt aaacaccagt ttcccagcaa 2820

acagcagcca aaggatgtgc catacaaaga actcttaaag cagctgactt cccagcagca 2880

tgcgattctt attgaccttg ggcgaacctt tcctacacac ccatacttct ctgcccagct 2940

tggagcagga cagctatcgc tttacaacat tttgaaggcc tactcacttc tagaccagga 3000

agtgggatat tgccaaggtc tcagctttgt agcaggcatt ttgcttcttc atatgagtga 3060

ggaagaggcg tttaaaatgc tcaagtttct gatgtttgac atggggctgc ggaaacagta 3120

tcggccagac atgattattt tacagatcca gatgtaccag ctctcgaggt tgcttcatga 3180

ttaccacaga gacctctaca atcacctgga ggagcacgag atcggcccca gcctctacgc 3240

tgccccctgg ttcctcacca tgtttgcctc acagttcccg ctgggattcg tagccagagt 3300

ctttgatatg atttttcttc agggaacaga ggtcatattt aaagtggctt taagtctgtt 3360

gggaagccat aagcccttga ttctgcagca tgaaaaccta gaaaccatag ttgactttat 3420

aaaaagcacg ctacccaacc ttggcttggt acagatggaa aagaccatca atcaggtatt 3480

tgaaatggac atcgctaaac agttacaagc ttatgaagtt gagtaccacg tccttcaaga 3540

agaacttatc gattcctctc ctctcagtga caaccaaaga atggataaat tagagaaaac 3600

caacagcagc ttacgcaaac agaaccttga cctccttgaa cagttgcagg tggcaaatgg 3660

taggatccaa agccttgagg ccaccattga gaagctcctg agcagtgaga gcaagctgaa 3720

gcaggccatg cttaccttag aactggagcg gtcggccctg ctgcagacgg tggaggagct 3780

gcggcggcgg agcgcagagc ccagcgaccg ggagcctgag tgcacgcagc ccgagcccac 3840

gggcgactga cagctctgca ggagagattg caacaccatc ccacactgtc caggccttaa 3900

ctgagaggga cagaagacgc tggaaggaga gaaggaagcg ggaagtgtgc ttctcaggga 3960

ggaaaccggc ttgccagcaa gtagattctt acgaactcca acttgcaatt cagggggcat 4020

gtcccagtgt tttttttgtt gtttttagat actaaatcgt cccttctcca gtcctgatta 4080

ctgtacacag tagctttaga tggcgtggac gtgaataaat gcaacttatg ttttcttgtt 4140

ggttcctttt tgagtgtcac tgtgtttgta aagagcattc acaatacggt ggaatttcaa 4200

aagctggaag agctcgagat catgcctcag gcaaaggcgt gggtccatcg ttcttccgag 4260

agggtttgtg tggcgactac accctcagcg tccctggcaa ggtgcagttg gctctcgccc 4320

attcttgtta tggaaaccta agatgatcat tgggaagatc agtgatcttg ggtcattgat 4380

ccctggctca gaggatagcg gtttccatca taaaccaaga tgatgagttc agcctttatc 4440

cctcgtggtt ccactagatg taacttaaag gagttaacat ttgaggactt tgttctacat 4500

cagattttac tatttgaatg tttaagatca ctttattgaa tttgaagatc atcaaattaa 4560

ataaaatgat ttatttaatt tggatatcct gatcactgtc aagtgaaatg gatctctctc 4620

tttggtattt aaggaagttt gtctttaaaa aaaaaataga gtgttttcat acatttttgc 4680

ttatcccata agtacagttg atcaaagtca tagtaggtaa atgctttatg ggacagctga 4740

caccttttag accctaccag gtattgctag catgtgagct gcagttgtgg ggtctgagat 4800

atttctttgt ggtagtttca tacccatact atagagtcat gtatttattt ttgcctgttg 4860

tgtgatgtaa tgcaatcatg ttcctttgag tctccatccc ttggaaatct gacttcttgc 4920

agaaggagta ggcacatcaa gatattcagg ggtgccccaa gagtctggga ctttcaaaaa 4980

aaaaagatca ggctgaaact gcagtcagat ttatgacagc tgacagtttt tcagaggtcg 5040

cacacagtga ctctcctctc tcaggatgac gaggacctgt gccttcaaca agcaaaatgc 5100

tgctcacggt tgtcctgctt gcagccagtc actgtgtaaa gcctctctga tgtgcactta 5160

agagtgggtt gctttctcac aaagatgggg ttctgtgcag tcacaggtca cttccttgac 5220

aacacaatca tttctgatct ttatcactgt aaccacgtct tctattccat aggagtttct 5280

tttgattctc tcagttgcgg ggggcatctc ttaatcctgg ggtaaaagga gagattgcca 5340

tacttagact cactgtgagt ctccccggcc atttcacgag gagaccacag tgctgccacc 5400

agtgcctaaa caggtggctg gcattcgaga cttcctcctg ttccctgggt cagaggatag 5460

cggtttccat cataaaccaa gatgatgagt tcagccttta tccctcgtgg ttccgctaga 5520

tgtaacttat aggagttaac atttgaggac tttgttctgc atcagatctt actatttgaa 5580

tgtttactgt tggattttgg gcatcttatt actgttactc aaaaacattg actctgcatc 5640

aagaaagaaa caagaaagca ataaaacaag aaataattca tgctcaca 5688

<210> 4
<211> 6465
<212> DNA
<213> Homo sapiens

<400> 4
ggccagagat gggtttagta gaggctaatc cgcggagggc acttcatccc gggtgtttct 60

ctgagtcccc gattttgctg cctggagtgc cagcgtccca tcctgcttcg gttcctggtt 120

ccccccacca gcccccatcc tgccattcga ccccaatgca agaaggatga ggcgggggaa 180

gggctggtgg ctgggtggcc tgcgggtacg ctgcgcagtg atctccgggt ccgtttcgga 240

gggtgcgtgc agagaagcag gatccctcag ggagcgcggg ctccgctgcc acctgctgag 300

aacctcgcac gccaaaacag cctcgggcag ggctacctgc tataaataag cggtcaagag 360

cccggtgtgt gaccacacat cccgccggga gggccataat cacttgtgag cacgcaggga 420

atattccggg ccaggacttc gcgctgaggc tggatggggt ggcgatgctt gcaccttccc 480

cgagctctcg tggggtggcg cctggtggcc cctgggcggg ctgaggatgg accgctgggg 540

agcgagggga gggaggccgc ggaggacccg cactgcagca gctgttgctc gcctgtgact 600

cgccagtctg ggccgagtcg cctagccaga gtcgcttggt gcggagggga agggagggca 660

tctactcctg ttcttggctc ccgggggact cactagggag attccggctg ctgtccccct 720

cccccgcctc tgctttcccc agcgctcgcc cagcccggga ggaggaaacg cgagcgagcc 780

gcgcgcagac acctcctcct cctcctcagc tgctgtcgcc gccgccggcc acaccgcggt 840

gcctgttgct gcaagccacc tcggaccgac cgtctcttca acccgcaaga gaagcagaga 900

ctgtggagtg gacccccacc ccagcagcgt ggggcggcaa gagcacagcc agctgaggac 960

catggacgcc ccgcccgccc aggccatctt ctgggtgccc gggagcacct gcgcgcccta 1020

gctcagcccg gagttctgag cgtgccacct cactggattc tgccctcaga agcacgtgaa 1080
atagatcact tcatttaaag taggaaaata gtagcacgtt ttcctggctc tccagacctc 1140
tcccaggatg gaggcaatca cattcacagc gaggaagcat ccgttcccta acgaagtctc 1200
cgtggacttc ggcttgcagc tggtgggctc cttacccgtg cattctctca ccactatgcc 1260
catgctgccg tgggtggtag ccgaggtacg aagactcagt ggccagtgct ctaaaaagga 1320
gcctaggaca aagcaagtcc gcctttgggt ttcgccctcc ggactgcggt gcgagcctga 1380
cctggagaaa agtcaaccat gggacccgct catctgttcc agcatctttg agtgcaagcc 1440
tcagcgtgtg cacaaactga ttcacaacag tcacgacccg agctactttg cttgcctcat 1500
taaggaggat gctgcgcaca ggcagagcct ctgctatgtg tttaaagcag acgatcaaac 1560
aaaagtgcct gagatcatca gctccatccg gcaggccggg aagattgccc gccaggaaga 1620
gctgcgttgc ccctccgagt tcgacgatac cttcgccaaa aagttcaagg tgctcttctg 1680
tggccgggtg actgtggctc acaagaaggc cccacccgca ctgattgacg agtgtatcga 1740
gaagttcaac catgtgagct gtggtcgcag aacggactgg gaagcgccca ccgggcagcc 1800
atcagcgcct ggccccaggc ccatgcgcaa atccttctca cagcctggac tgcgctcgct 1860
ggccttcagg aaggagttcc aggacgctag cctccgcagt agcaccttta gctcctttga 1920
caatgacata gagaaccacc tcatcggtgg gcacaatgtg gttcagccca cagacatgga 1980
ggagaaccga actatgctgt tcacgattgg ccaatctgaa gtttacctca tcagtcctga 2040
caccaaaaag attgcactgg agaaaaattt taaggagata tccttttgct ctcagggcat 2100
cagacatgtg gaccactttg gattcatctg ccgagagtgc tcgggtggcg gcagtggcgg 2160
ctttcatttt gtctgttacg tgttccagtg cacaaatgaa gctctggttg acgagatcat 2220
gatgactctg aagcaggctt tcacggtagc tgcggtgcag cagacggcta aggcaccagc 2280
ccagctctgt gagggctgcc ccttgcaagg cctgcacaag ctctgcgaaa ggatagaggg 2340
aatgaattca tctaaaacca aattagaact ccagaagcac ttgaccacac tgaccaatca 2400
ggagcaggcc accatattcg aggaggttca gaaattgaga ccaagaaacg agcagcgaga 2460
gaatgaatta attatttctt ttctgaggtg cttatatgaa gagaagcaaa aagagcacag 2520
ccacactggg gagccaaagc agacactaca ggtggcagca gagaatattg ggagtgacct 2580
gccacccagt gctagccggt tcaggttaga ttcgctgaag aacagagcaa agaggtcctt 2640
aacagagtcc ctagagagca ttctgtcccg gggtaataaa gccagaggcc tgcaggacca 2700
ttccgccagt gtggatctgg acagctccac ttctagtact ctaagtaaca ccagcaaaga 2760
gctgtccatg ggtgacaagg aggccttccc cgtctctgag acctccttca agctccttgg 2820
ctcctcagat gacctgtcca gtgactcaga gggccacatt gcagaagagt ctgccctgtt 2880

gtcaccccag caggcgttca gaaggagagc caacaccctg agtcatttcc cagtagagtg 2940

ccctgcgcct ccagaacctg cccagagctc tccaggggtc tctcaaagga aactcatgcg 3000

gtaccactcc gtgagcacag agacgcctca tgaacgcaat gtggaccatc ttcctggggg 3060

tgagtctcag ggctgcccag ggcagccttc agctccgcct ccacctcgtc ttaacccctc 3120

cgcctcctca ccaaacttct ttaagtacct aaaacacaat tccagtggag agcaaagtgg 3180

gaatgctgtg ccaaaaagtg tctcctaccg taatgccctg cgaaaaaaac tccattcttc 3240

ctcctctgtg ccaaattttc taaaatttct ggctcccgta gatgaaaata acacctgtga 3300

ctttaagaac acgaacaggg actttgaatc caaagcaaac cacctgggtg acacagatgg 3360

gacccccgtg aagacccggc ggcactcgtg gagacagcag atattccttc gagtggccac 3420

tccacagaag gcttgtgact ccccgagcag atatgaagat tattccgagc tgggagagct 3480

ccctccacgc tcccctttag aaccggtgtg tgaggacggc ccatttggcc cagtacagga 3540

agaaaagagg aagacgtcac gcgagcttcg agagctgtgg aaaaaggcca tcttgcagca 3600

gatcctgctg ctcaggatgg agaaggagaa tcagaagcta caagcctctg aaaacgattt 3660

gctgaacaaa cgcctcaagc ttgactatga agaaatcact ccgtgtctta aagaagtcac 3720

tacagtgtgg gaaaagatgc ttagcactcc aggaagatcc aaaattaagt ttgacatgga 3780

aaaagtgcac tcagctgttg ggcaaggtgt gccacgtcat caccgaggtg agatctggaa 3840

atttctagct gagcagttcc accttaaaca cccatttcct agtaaacagc agccaaagga 3900

cgtgccctac aaagagctcc tgaagaagct gacctcgcag cagcacgcca ttctcatcga 3960

cctcgggcga acctttccaa cacatccata cttctctgcc cagcttggag caggtcagct 4020

gtcactttac aacattctga aggcctactc gcttctggac caggaggttg gatactgcca 4080

aggtctcagc tttgtggcag gcattttgct tcttcacatg agtgaggaag aggcgttcaa 4140

gatgctcaag ttcctgatgt ttgacatggg gctgcggaaa cagtatcggc cagacatgat 4200

tattttgcag atccagatgt accagctgtc acggctcctc cacgattacc accgagacct 4260

ctacaaccac ctggaagagc acgagatcgg ccccagcctc tacgcggctc cctggtttct 4320

caccgtgttc gcctcacagt tcccactcgg ctttgtagcc agagtctttg atatgatctt 4380

ccttcaggga tcagaggtca tatttaaagt agctttaagt cttttgggga gccataagcc 4440

cttgattcta cagcatgaga acctggaaac catcgtggac ttcataaaga acacactccc 4500

caacctgggc ctggtgcaga tggagaagac catcagtcag gtgtttgaga tggacatcgc 4560

caagcagctc caggcctatg aggtcgagta ccacgtgctc caggaggagc ttattgagtc 4620

ctcgcctctc agtgacaacc aaagaatgga gaaattggag aaaaccaaca gcagcttgcg 4680

caaacagaac cttgacctcc tggagcagtt gcaggtggca aatgctagga tccaaagcct 4740

tgaagccacg gtagagaaac ttcttaccag cgagagtaag ctgaagcagg ctgcgctgac 4800

cctggaggtg gagcgctccg ccctgctgca gatggtggag gagctgcgga ggcaaagcgc 4860

ccggcccagc actccagagc cagactgcac ccagctggag cccacaggcg attgaccgct 4920

gccagaagag actgtgcacc attaacactg tccaagcctt aatcaagaga gatggaagtc 4980

agaggcagag aagagagaac ttctcaggga ggaaactggc tgaccagcct gcagatcctt 5040

ttgagctcag aacttgggat tggaggacaa aagtctcaga gttattgttg tttttggttc 5100

taatccgtcc cctttccagt cctggttgtt gtagctttag atggcatgga catgaataaa 5160

ttacatttat ggttcctcct ccttcttgca gagacacagt gtgtggaaag agcattcatg 5220

actgcagtgt tccaaaaaca agatgggtct gaaataattc ctcaggcaga agaaacgtga 5280

ccctttgtca gtctgcaaga aaggttcctg ggagggtgcc cttgatgtca cctgaggtgc 5340

agtgggccct tgtccatttt tattatggga cctaaggtga ccatgaggaa ggaagaaagg 5400

ggtttattta aatcagatat attggttgtt cctgaatcca cggtcactcc cattggtact 5460

gaagagagct catcttttac aaactcagtg ttttagtatg gtttttgctt accctttcaa 5520

gtcacaggta aatgtttcat aaggaaatca cagtttttag attccctgtt attgctagtg 5580

tgggacctgt agctgtgggg tctgacatat ttatttcttt ttgattgttt catatccaca 5640

ctatagggtc acatatttat ttttgtctgt tgataaatga tgtaccactg ccacatcttc 5700

ctttgagcct tgaacccctt ggaaatctga tttctcatag aaggaacaaa acataccaag 5760

atttccaggg gctcctaaga tgctgaaaca ttgcaaaaga aaccagattg caatcaggtg 5820

tatggcagct gacagctttt cagaagtagg aagtgtgttt tgaaagtact caaacacacg 5880

cacgccttcc ttgggatggc gaggaccctg tgccttcaag cagagtgcag ctcacactca 5940

ccctgcttct agctggtcct gtgtagagca tcacccatgt gtgcaacagc tttttttttt 6000

cttttaacat acacacaaag gcttaggtta gggaagacca ccagccagga gccctggcta 6060

tcggaattgg tcctgccttg ggctctgttg gccctcctgt tcctggaggt ctgctgcagc 6120

gctgttggta ggaaaggagc cttgtaagtc tgggctgtaa ggagagacca tcaccgttgg 6180

ggtcatttgg gatctcctag ccctatcaaa gggacactaa atggccagga gggatgtaag 6240

actcccacat ggtcttgggt cagaggagag gtctctccat aaaccaacat gcccagtccc 6300

agttggttgg agcaggggtt gtcagaggag ggtgacagtg gacagcttct ttccacattg 6360

tattttatta tttgaatgtt ttaatgttga attttggaca tgttattact gttcaaaaaa 6420

cactgactct acagtaagaa aagagcaata aaacagatta ttcac 6465

<210> 5
<211> 30
<212> DNA
<213> Mus musculus

<400>  5
tgcgtcgacg aagcagagac tgtggagtgg                                        30


<210>  6
<211>  29
<212>  DNA
<213>  Mus musculus

<400>  6
atcgtataca acaaccagga ctggaaagg                                         29


<210>  7
<211>  19
<212>  DNA
<213>  Mus musculus

<400>  7
agtggccact ccacagaag                                                    19


<210>  8
<211>  19
<212>  DNA
<213>  Mus musculus

<400>  8
tcctgtactg ggccaaatg                                                    19


<210>  9
<211>  35
<212>  DNA
<213>  Mus musculus

<400>  9
actccccgag cagatatgaa gattattccg agctg                                  35


<210>  10
<211>  19
<212>  DNA
<213>  Mus musculus

<400>  10
atgtggacca tcttcctgg                                                    19


<210>  11
<211>  19
<212>  DNA
<213>  Mus musculus

<400>  11
gctctccact ggaattgtg                                                    19


<210>  12
<211>  27
<212>  DNA
<213>  Mus musculus

<400>  12
ttcagctccg cctccacctc gtcttaa                                          27

<210>  13
<211>  21
<212>  DNA
<213>  Mus musculus

<400>  13
gagcaggtca gctgtcactt t                                                21

<210>  14
<211>  20
<212>  DNA
<213>  Mus musculus

<400>  14
tgagaccttg gcagtatcca                                                  20

<210>  15
<211>  25
<212>  DNA
<213>  Mus musculus

<400>  15
aaaggttaaa tctggatctt gagtg                                            25

<210>  16
<211>  22
<212>  DNA
<213>  Mus musculus

<400>  16
ctcctcaagt ttgttttgct ca                                               22

<210>  17
<211>  27
<212>  DNA
<213>  Mus musculus

<400>  17
tataaagata atcagggctt aaactcg                                          27

<210>  18
<211>  20
<212>  DNA
<213>  Mus musculus

<400>  18
acctccacaa catgagcaca                                                  20

<210>  19
<211>  27
<212>  DNA
<213>  Mus musculus

<400>  19
tttaagttgt gttaatgttg aaagttc                                          27


<210>  20
<211>  20
<212>  DNA
<213>  Mus musculus

<400>  20
cacacatgca cgaacatgta                                                  20


<210>  21
<211>  19
<212>  DNA
<213>  Mus musculus

<400>  21
gcatgtgcgt gtgcttatg                                                   19


<210>  22
<211>  24
<212>  DNA
<213>  Mus musculus

<400>  22
aatgtatacc tccacaaaca cctc                                             24


<210>  23
<211>  26
<212>  DNA
<213>  Mus musculus

<400>  23
tagatgctgc ctaacaatta gtaagt                                           26


<210>  24
<211>  24
<212>  DNA
<213>  Mus musculus

<400>  24
tgggaatatt ttttcaagtg tctc                                             24


<210>  25
<211>  20
<212>  DNA
<213>  Mus musculus

<400>  25
agaattggga cgaagccttt                                                  20


<210>  26
<211>  25
<212>  DNA
<213>  Mus musculus

<400>  26
tttgaaatca agattagcca ttacc                                          25


<210>  27
<211>  25
<212>  DNA
<213>  Mus musculus

<400>  27
aaaatcagtt aaggtagcac aatgc                                          25


<210>  28
<211>  18
<212>  DNA
<213>  Mus musculus

<400>  28
gttggccatc tttgctgg                                                  18


<210>  29
<211>  21
<212>  DNA
<213>  Mus musculus

<400>  29
ccctgtgctc tggattagtt g                                              21


<210>  30
<211>  20
<212>  DNA
<213>  Mus musculus

<400>  30
tcaagaccag catcaaacca                                                20


<210>  31
<211>  20
<212>  DNA
<213>  Mus musculus

<400>  31
gtgcaggcct gaattgaaat                                                20


<210>  32
<211>  19
<212>  DNA
<213>  Mus musculus

<400>  32
caaagtgcct gtgcatgtg                                                 19


<210>  33
<211>  26
<212>  DNA
<213>  Mus musculus

<400>  33
aagagagaca tacacaaaca gacaca                                          26


<210>  34
<211>  27
<212>  DNA
<213>  Mus musculus

<400>  34
tttcttaaca agtttctagc aatttcc                                         27


<210>  35
<211>  27
<212>  DNA
<213>  Mus musculus

<400>  35
tgtctgtctg tcattctgtc atatatg                                         27


<210>  36
<211>  22
<212>  DNA
<213>  Mus musculus

<400>  36
ccaaaaacca gatttgagat cc                                              22


<210>  37
<211>  22
<212>  DNA
<213>  Mus musculus

<400>  37
aagaccactt gaaaacagtg ca                                              22


<210>  38
<211>  24
<212>  DNA
<213>  Mus musculus

<400>  38
ggagatctaa atttcaaaac acca                                            24


<210>  39
<211>  27
<212>  DNA
<213>  Mus musculus

<400>  39
cattttaaaa cagaaatctg aagttcc                                         27


<210>  40
<211>  22
<212>  DNA
<213>  Mus musculus

<400> 40
gtgactaggt atgccaaatc cc 22

<210> 41
<211> 27
<212> DNA
<213> Mus musculus

<400> 41
tcatgactat aaatgtattc atgtgtg 27

<210> 42
<211> 23
<212> DNA
<213> Mus musculus

<400> 42
cttcaaagac tcccatatac ccc 23

<210> 43
<211> 21
<212> DNA
<213> Mus musculus

<400> 43
cacagagaag aatctgcgag c 21

<210> 44
<211> 26
<212> DNA
<213> Mus musculus

<400> 44
ttgcaaagtg ttttaatcag tatttg 26

<210> 45
<211> 26
<212> DNA
<213> Mus musculus

<400> 45
ggttaagaaa aagacagaaa atctgc 26

<210> 46
<211> 26
<212> DNA
<213> Mus musculus

<400> 46
ggttaagaaa aagacagaaa atctgc 26

<210> 47
<211> 19
<212> DNA
<213> Mus musculus

<400>  47
   acccctattc agtgccagg                                                    19


<210>  48
<211>  19
<212>  DNA
<213>  Mus musculus

<400>  48
acccaggcca caaaagaac                                                     19


<210>  49
<211>  26
<212>  DNA
<213>  Mus musculus

<400>  49
tcagtgataa tgacatggta gaagtg                                             26


<210>  50
<211>  18
<212>  DNA
<213>  Mus musculus

<400>  50
agagggtttc cttccccc                                                      18


<210>  51
<211>  18
<212>  DNA
<213>  Mus musculus

<400>  51
tcaaaaccca gcctcctg                                                      18


<210>  52
<211>  23
<212>  DNA
<213>  Mus musculus

<400>  52
agagtgtgac taacaggtga ggc                                                23


<210>  53
<211>  20
<212>  DNA
<213>  Mus musculus

<400>  53
ccatctctcc agcccctaga                                                    20


<210>  54
<211>  20
<212>  DNA
<213>  Mus musculus

<400> 54
ctatgggcac cagtcagaca 20

<210> 55
<211> 20
<212> DNA
<213> Mus musculus

<400> 55
ggcaacacaa aatggactca 20

<210> 56
<211> 20
<212> DNA
<213> Mus musculus

<400> 56
ccaacatctc ctgtgtccct 20

<210> 57
<211> 22
<212> DNA
<213> Mus musculus

<400> 57
tcagtttgat tgggtttctt cc 22

<210> 58
<211> 21
<212> DNA
<213> Mus musculus

<400> 58
cacacataca tgcactcctg c 21

<210> 59
<211> 21
<212> DNA
<213> Mus musculus

<400> 59
accccatctc tgtctctctg a 21

<210> 60
<211> 18
<212> DNA
<213> Mus musculus

<400> 60
ctgggtgaga ggggaggt 18

<210> 61
<211> 21
<212> DNA
<213> Mus musculus

<400>  61
ttagcccact gtagcatcac c                                                21

<210>  62
<211>  26
<212>  DNA
<213>  Mus musculus

<400>  62
tatttttcac acaagaactt actggg                                           26

<210>  63
<211>  21
<212>  DNA
<213>  Mus musculus

<400>  63
tacctaccac aatgctctgg g                                                21

<210>  64
<211>  20
<212>  DNA
<213>  Mus musculus

<400>  64
tcccattgag ccttttagga                                                  20

<210>  65
<211>  27
<212>  DNA
<213>  Mus musculus

<400>  65
ctgaaaaaaa aagttctcta ttcttga                                          27

<210>  66
<211>  23
<212>  DNA
<213>  Mus musculus

<400>  66
tgagcagaaa gtctattcca gaa                                              23

<210>  67
<211>  22
<212>  DNA
<213>  Mus musculus

<400>  67
cattttcatc ttattgccca tg                                               22

<210>  68
<211>  24
<212>  DNA
<213>  Mus musculus

<400>  68
acctaaagac agatacagga accc                                              24

<210>  69
<211>  25
<212>  DNA
<213>  Mus musculus

<400>  69
attatcagcc atcttcattc ataca                                             25

<210>  70
<211>  27
<212>  DNA
<213>  Mus musculus

<400>  70
gcctcacctt taaacaatac tactaga                                           27

<210>  71
<211>  21
<212>  DNA
<213>  Mus musculus

<400>  71
aagatgggaa aatcagggat g                                                 21

<210>  72
<211>  23
<212>  DNA
<213>  Mus musculus

<400>  72
aaatgttccc ttcattttct tcc                                               23

<210>  73
<211>  22
<212>  DNA
<213>  Mus musculus

<400>  73
gcataaaatg gatttcctga gg                                                22

<210>  74
<211>  20
<212>  DNA
<213>  Mus musculus

<400>  74
tgtcccaaaa caaacaaccc                                                   20

<210>  75
<211>  20
<212>  DNA
<213>  Mus musculus

<400>  75
aagccagaag cctgttgtgt                                                    20


<210>  76
<211>  23
<212>  DNA
<213>  Mus musculus

<400>  76
gtctgttgtg agttcagtag ggg                                                23


<210>  77
<211>  20
<212>  DNA
<213>  Mus musculus

<400>  77
ccatgcccag tctggtatct                                                    20


<210>  78
<211>  24
<212>  DNA
<213>  Mus musculus

<400>  78
ttttactttc cactaagcat tccc                                               24


<210>  79
<211>  21
<212>  DNA
<213>  Mus musculus

<400>  79
ccttccacat acataggcag g                                                  21


<210>  80
<211>  20
<212>  DNA
<213>  Mus musculus

<400>  80
cctcagtgct ggatggattt                                                    20


<210>  81
<211>  20
<212>  DNA
<213>  Mus musculus

<400>  81
tacacaggga aaggacaggg                                                    20


<210>  82
<211>  22
<212>  DNA
<213>  Mus musculus

<400> 82
agggagattt tggattagag gc 22

<210> 83
<211> 20
<212> DNA
<213> Mus musculus

<400> 83
gaggactcct tcgatttccc 20

<210> 84
<211> 21
<212> DNA
<213> Mus musculus

<400> 84
ttcctaagca ggaactgacc a 21

<210> 85
<211> 24
<212> DNA
<213> Mus musculus

<400> 85
acttcttgtt actgcatgtg aagc 24

<210> 86
<211> 22
<212> DNA
<213> Mus musculus

<400> 86
caagagagca gagttagtgg ca 22

<210> 87
<211> 25
<212> DNA
<213> Mus musculus

<400> 87
agtggcataa cttcattcaa taatg 25

<210> 88
<211> 27
<212> DNA
<213> Mus musculus

<400> 88
ggaatcaagt tgtttttaa atttacc 27

<210> 89
<211> 20
<212> DNA
<213> Mus musculus

<400>  89
agggcagcag caacaaatac                                                    20


<210>  90
<211>  27
<212>  DNA
<213>  Mus musculus

<400>  90
tcatcgtata aatgtatcaa ttctcaa                                            27


<210>  91
<211>  25
<212>  DNA
<213>  Mus musculus

<400>  91
agcttttact gaaatgtttt gtagg                                              25


<210>  92
<211>  20
<212>  DNA
<213>  Mus musculus

<400>  92
tgagcaggag cctatttgtg                                                    20


<210>  93
<211>  20
<212>  DNA
<213>  Mus musculus

<400>  93
cccttcagag ccaagagaaa                                                    20


<210>  94
<211>  20
<212>  DNA
<213>  Mus musculus

<400>  94
ttcctttgcc tctgtcacct                                                    20


<210>  95
<211>  20
<212>  DNA
<213>  Mus musculus

<400>  95
cacgcctcca ttagaacctc                                                    20


<210>  96
<211>  22
<212>  DNA
<213>  Mus musculus

<400> 96
tttcatggat ctcttaggca ca                                              22

<210> 97
<211> 20
<212> DNA
<213> Mus musculus

<400> 97
cgctgaattc ttcccaagtg                                                 20

<210> 98
<211> 22
<212> DNA
<213> Mus musculus

<400> 98
ggtcatctct aggcaggatg tt                                              22

<210> 99
<211> 22
<212> DNA
<213> Mus musculus

<400> 99
ggtcaagtga aatcttcctg ct                                              22

<210> 100
<211> 21
<212> DNA
<213> Mus musculus

<400> 100
ccagttaagc atttccccct a                                               21

<210> 101
<211> 20
<212> DNA
<213> Mus musculus

<400> 101
gccctgcaga tagacgcttt                                                 20

<210> 102
<211> 20
<212> DNA
<213> Mus musculus

<400> 102
acccaaggtt ttctggctct                                                 20